# EUROPEAN PATENT APPLICATION

(11) **EP 2 128 158 A1**
(43) Date of publication of application: **02.12.2009**
(21) Application number: 08009576.3
(22) Date of filing: 26.05.2008
(51) Int. Cl.: C07D 413/12, A61K 31/4439, A61P 1/16, A61P 3/10, A61P 7/02, A61P 9/10

(54) **Heterocyclic cyclopropyl-substituted FXR binding compounds**

(71) Applicant: Phenex Pharmaceuticals AG, 67056 Ludwigshafen (DE)
(72) Inventor: Abel, Ulrich, 69126 Heidelberg (DE); Kremoser, Claus, 69121 Heidelberg (DE)
(74) Representative: Grünecker, Kinkeldey, Stockmair & Schwanhäusser Anwaltssozietät

(57) **Abstract**

The present invention relates to compounds defined by formula (I): 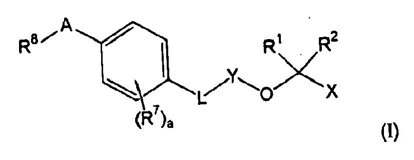 or an enantiomer, diastereomer, tautomer, solvate or pharmaceutically acceptable salt thereof,
wherein
R¹ and R² are independently from each other selected from hydrogen, fluorine and C₁-C₃ alkyl;
X is 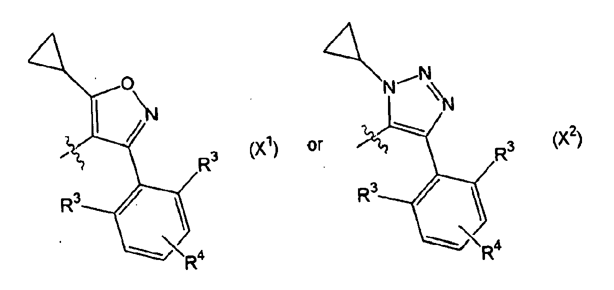 which bind to the NR1H4 receptor (FXR) and act as agonists of the NR1H4 receptor (FXR). The invention further relates to the use of the compounds for the preparation of a medicament for the treatment of diseases and/or conditions through binding of said nuclear receptor by said compounds, such as the prophylaxis and/or treatment of chronic intrahepatic or some forms of extrahepatic cholestatic conditions, liver fibrosis resulting from chronic cholestatic conditions, acute intraheptic cholestatic conditions, obstructive or chronic inflammatory disorders that arise out of improper bile composition, gastrointestinal conditions with a reduced uptake of dietary fat and fat-soluble dietary vitamins, inflammatory bowel diseases, lipid and lipoprotein disorders, Type II Diabetes, clinical complications of Type I and Type II Diabetes.

## Description

The present invention relates to compounds which bind to the NR1H4 receptor (FXR) and act as agonists of the NR1H4 receptor (FXR). The invention further relates to the use of the compounds for the treatment and/or prophylaxis of diseases and/or conditions through binding of said nuclear receptor by said compounds.

Multicellular organisms are dependent on advanced mechanisms of information transfer between cells and body compartments. The information that is transmitted can be highly complex and can result in the alteration of genetic programs involved in cellular differentiation, proliferation, or reproduction. The signals, or hormones, are often low molecular weight molecules, such as peptides, fatty acid, or cholesterol derivatives.

Many of these signals produce their effects by ultimately changing the transcription of specific genes. One well-studied group of proteins that mediate a cell's response to a variety of signals is the family of transcription factors known as nuclear receptors, hereinafter referred to often as "NR". Members of this group include receptors for steroid hormones, vitamin D, ecdysone, cis and trans retinoic acid, thyroid hormone, bile acids, cholesterol-derivatives, fatty acids (and other peroxisomal proliferators), as well as so-called orphan receptors, proteins that are structurally similar to other members of this group, but for which no ligands are known. Orphan receptors may be indicative of unknown signalling pathways in the cell or may be nuclear receptors that function without ligand activation. The activation of transcription by some of these orphan receptors may occur in the absence of an exogenous ligand and/or through signal transduction pathways originating from the cell surface (D. Mangelsdorf et al. "The nuclear receptor superfamily: the second decade", Cell 1995, 83(6), 835-839; R Evans "The nuclear receptor superfamily: a rosetta stone for physiology" Mol. Endocrinol. 2005, 19(6), 1429-1438).

In general, three functional domains have been defined in NRs. An amino terminal domain is believed to have some regulatory function. A DNA-binding domain hereinafter referred to as "DBD" usually comprises two zinc finger elements and recognizes a specific Hormone Responsive Element hereinafter referred to as "HRE" within the promoters of responsive genes. Specific amino acid residues in the "DBD" have been shown to confer DNA sequence binding specificity (M. Schena "Mammalian glucocorticoid receptor derivatives enhance transcription in yeast", Science 1988, 241 (4868), 965-967). A ligand-binding-domain hereinafter referred to as "LBD" is at the carboxy-terminal region of known NRs.

In the absence of hormone, the LBD appears to interfere with the interaction of the DBD with its HRE. Hormone binding seems to result in a conformational change in the NR and thus opens this interference (A. Brzozowski et al. "Molecular basis of agonism and antagonism in the oestrogen receptor" Nature 1997, 389(6652), 753-758). A NR without the LBD constitutively activates transcription but at a low level.

Coactivators or transcriptional activators are proposed to bridge between sequence specific transcription factors, the basal transcription machinery and in addition to influence the chromatin structure of a target cell. Several proteins like SRC-1, ACTR, and Grip1 interact with NRs in a ligand enhanced manner (D. Heery et al. "A signature motif in transcriptional co-activators mediates binding to nuclear receptors" Nature 1997, 387(6634), 733-6.; T. Heinzel et al. "A complex containing N-CoR, mSin3 and histone deacetylase mediates transcriptional repression" Nature 1997, 387(6628), 16-17; K. Nettles, G. Greene "Ligand control of coregulator recruitment to nuclear receptors" Annu. Rev. Physiol. 2005, 67, 309-33).

Nuclear receptor modulators like steroid hormones affect the growth and function of specific cells by binding to intracellular receptors and forming nuclear receptor-ligand complexes. Nuclear receptor-hormone complexes then interact with a hormone response element (HRE) in the control region of specific genes and alter specific gene expression (A. Aranda, A. Pascual "Nuclear hormone receptors and gene expression" Physiol. Rev. 2001, 81(3), 1269-1304).

The Farnesoid X Receptor alpha (hereinafter also often referred to as NR1H4 when referring to the human receptor) is a prototypical type 2 nuclear receptor which activates genes upon binding to promoter region of target genes in a heterodimeric fashion with Retinoid X Receptor (B. Forman et al. "Identification of a nuclear receptor that is activated by farnesol metabolites" Cell 1995, 81(5), 687-693). The relevant physiological ligands of NR1H4 are bile acids (D. Parks et al. "Bile acids: natural ligands for an orphan nuclear receptor" Science 1999, 284(5418), 1365-1368; M. Makishima et al. "Identification of a nuclear receptor for bile acids" Science 1999, 284(5418), 1362-1365). The most potent one is chenodeoxycholic acid (CDCA), which regulates the expression of several genes that participate in bile acid homeostasis. Farnesol and derivatives, together called farnesoids, are originally described to activate the rat orthologue at high concentration but they do not activate the human or mouse receptor. FXR is expressed in the liver, small intestine, colon, ovary, adrenal gland and kidney. Beyond controlling intracellular gene expression, FXR seems to be also involved in paracrine and endocrine signaling (J. Holt et al. "Definition of a novel growth factor-dependent signal cascade for the suppression of bile acid biosynthesis" Genes Dev. 2003, 17(13), 1581-91; T. Inagaki et al. "Fibroblast growth factor 15 functions as an enterohepatic signal to regulate bile acid homeostasis" Cell Metab. 2005, 2(4), 217-225).

There is one publication which proposes a direct impact of FXR activation on the survival of infectious organisms such as bacteria or protozoic parasites via the upregulation of the lysosomal fate/survival factor Taco-2 in macrophages (P. Anandet al. "Downregulation of TACO gene transcription restricts mycobacterial entry/survival within human macrophages" FEMS Microbiol. Lett. 2005, 250(1), 137-144). This might pave the way for further studies that assess the suitability of FXR to act as drug target for the treatment of intracellular bacterial or parasitic infections such as Tuberculosis, Lepra, Leishmaniosis or Trypanosomiasis, e.g. Chagas Disease.

Small molecule compounds which act as FXR modulators have been disclosed in the following publications: WO2008/051942, WO2008/025539, WO2008/025540, WO2007/140183, WO2007/092751, WO2007/140174, WO2007/140183, WO2004/048349, WO2003/015771 and WO2000/037077. Further small molecule FXR modulators have been recently reviewed (R. C. Buijsman et al. "Non-Steroidal Steroid Receptor Modulators" Curr. Med. Chem. 2005, 12, 1017-1075).

Many of the failures of drug candidates in development programs are attributed to their undesirable pharmacokinetic properties, such as too long or too short t_{1/2}, poor absorption, and extensive first-pass metabolism. In a survey, it was reported that of 319 new drug candidates investigated in humans, 77 (40%) of the 198 candidates were withdrawn due to serious pharmacokinetic problems (R. Prentis et al. "Pharmaceutical innovation by seven UK-owned pharmaceutical companies (1964-1985)" Br. J. Clin. Pharmacol. 1988, 25, 387-396). This high failure rate illustrates the importance of pharmacokinetics in drug discovery and development. To ensure the success of a drug's development, it is essential that a drug candidate has good bioavailability and a desirable t_{1/2}. Therefore, an accurate estimate of the pharmacokinetic data and a good understanding of the factors that affect the pharmacokinetics will guide drug design (J. Lin, A. Lu "Role of pharmacokinetics and Metabolism in Drug Discovery and Development" Pharmacol. Rev. 1997, 49(4), 404-449). Chemically modifiable factors that influence drug absorption and disposition are discussed as follows.

Some relevant physicochemical and ADME parameters include but are not limited to: aqueous solubility, logD, PAMPA permeability, Caco-2 permeability, plasma protein binding, microsomal stability and hepatocyte stability.

Poor aqueous solubility can limit the absorption of compounds from the gastrointestinal (GI) tract, resulting in reduced oral bioavailability. It may also necessitate novel formulation strategies and hence increase cost and delays. Moreover, compound solubility can affect other *in vitro* assays. Poor aqueous solubility is an undesired characteristic and it is the largest physicochemical problem hindering oral drug activity (C. A. Lipinski "Drug-like properties and the causes of poor solubility and poor permeability", J. Pharmacol. Toxicol. Methods 2000, 44, 235-249).

Lipophilicity is a key determinant of the pharmacokinetic behaviour of drugs. It can influence distribution into tissues, absorption and the binding characteristics of a drug, as well as being an important factor in determining the solubility of a compound. LogD (distribution coefficient) is used as a measure of lipophilicity. One of the most common methods for determining this parameter is by measuring the partition of a compound between an organic solvent (typically octanol) and aqueous buffer. An optimal range for lipophilicity tends to be if the compound has a logD value between 0 and 3. Typically, these compounds have a good balance between solubility and permeability and this range tends to be optimal for oral absorption and cell membrane permeation. Hydrophilic compounds (logD <0) typically are highly soluble but exhibit low permeability across the gastrointestinal tract or blood brain barrier. Highly lipophilic compounds (logD > 5) exhibit problems with metabolic instability, high plasma protein binding and low solubility which leads to variable and poor oral absorption (L. Di, E. Kerns "Profiling drug-like properties in discovery research" Curr. Opin. Chem. Biol. 2003, 7, 402-408).

Drug permeability through cell monolayers or artificial membranes correlates well with intestinal permeability and oral bioavailability. Drugs with low membrane permeability, i.e. low lipophilicity, are generally absorbed slowly from solution in the stomach and small intestine. Knowing the rate and extent of absorption across the intestinal tract is critical if a drug is to be orally delivered. Drug permeability cannot be accurately predicted by physicochemical factors alone because there are many drug transport pathways. A generally accepted human cell-based model, human colon adenocarcinoma cell line (Caco-2), helps to predict intestinal permeability (A.M. Marino et al. "Validation of the 96-well Caco-2 cell culture model for high-throughput permeability and assessment of discovery compounds", Int. J. Pharmaceutics 2005, 297; 235-241). This assay is commonly employed during early discovery, especially in lead optimisation. A newer *in vitro* model, known as the parallel artificial membrane permeability assay (PAMPA) ranks compounds on their passive diffusion rates alone. PAMPA is increasingly used as the first-line permeability screen during lead profiling (F. Wohnsland, B. Faller "High-throughput Permeability pH Profile and High-throughput Alkane/Water Log P With Artificial Membranes", J. Med. Chem. 2001, 44, 923-930).

Plasma protein binding (PPB) can significantly affect the therapeutic action of a drug. It determines the extent and duration of action because only unbound drug is thought to be available for passive diffusion to extravascular space or tissue sites where therapeutic effects occur. The level of PPB is important for predicting the pharmacokinetic profile of a drug and determining appropriate oral dosing. *In vivo* dose levels can be estimated from the determined fraction of unbound drug (fu); an increase in dose may be necessary if a drug is highly bound to plasma (Y. Kwon "Handbook of essential pharmacokinetics, pharmacodynamics and drug metabolism for industrial scientists" Springer Verlag 2001).

In vitro models to predict compound metabolism have become accepted adjuncts to animal testing. Early drug metabolism models help predict the metabolic stability of a compound and there are several approaches to doing this. The enzyme sources in these studies are rat or human derived systems that consist of liver microsomes and hepatocytes. Microsomes contain the full complement of phase I oxidative enzymes but do not have an intact cell membrane. Moreover, microsomes require the addition of a co-factor to the incubation. Hepatocytes are more representative of the *in vitro* situation because they contain a cell membrane and do not require additional cofactors. Hepatocytes contain enzymes for both phase I (oxidation, reduction and/or hydrolysis of test compound) and phase II (conjugation of test compounds or metabolites from phase I) metabolism. The microsomal stability screen is often used as a primary screen early in the drug discovery process. The hepatocyte stability assay is used as a secondary screen for the more favourable compounds discovered from the primary screen (T. Iwatsubo et al. "Prediction of in vivo drug metabolism in the human liver from in vitro metabolism data", Pharm. Ther. 1997, 73, 147-171).

In summary, favourable physicochemical and *in vitro* ADME parameters are prerequisite for a favourable pharmacokinetic (PK) profile of a drug. Obtaining early stage PK data evaluation of new chemical entities is a prerequisite for successful animal pharmacology and toxicology studies. Quantitative measures of drug exposure are key components needed for the sound interpretation of preclinical efficacy studies. PK data can also help in the design or species selection of preclinical toxicology studies. Pharmacokinetic studies are part of the regulatory drug development requirements and have also started to become an integral part of the early drug discovery process.

It is the object of the present invention to provide compounds that are FXR agonists which exhibit increased potency *in vitro* and increased plasma levels after oral administration *in vivo*, as compared to known FXR agonists described in the art.

Unexpectedly, we found that FXR agonists described herein exhibit increased potency towards FXR in biochemical as well as cellular *in vitro* assays compared to compounds described in the art.

Surprisingly, we also found that FXR agonists described herein exhibit increased plasma levels after oral administration *in vivo* as compared to known FXR agonists

As a result, the present invention relates to compounds according to the general formula (I) which bind to the NR1H4 receptor (FXR) and act as agonists of the NR1H4 receptor (FXR).

The invention further relates to the use of said compounds for the treatment and/or prophylaxis of diseases and/or conditions through binding of said nuclear receptor by said compounds. Specifically, the present invention relates to the use of compounds according to formula (I) in the preparation of a medicament for the prophylaxis and/or treatment of chronic intrahepatic or some forms of extrahepatic cholestatic conditions, liver fibrosis resulting from chronic cholestatic conditions, acute intraheptic cholestatic conditions, obstructive or chronic inflammatory disorders that arise out of improper bile composition, gastrointestinal conditions with a reduced uptake of dietary fat and fat-soluble dietary vitamins, inflammatory bowel diseases, lipid and lipoprotein disorders, Type II Diabetes, clinical complications of Type I and Type II Diabetes, conditions and diseases which result from chronic fatty and fibrotic degeneration of organs due to enforced lipid and specifically triglyceride accumulation and subsequent activation of profibrotic pathways, obesity and metabolic syndrome (combined conditions of dyslipidemia, diabetes and abnormally high body-mass index), acute myocardial infarction, acute stroke, thrombosis which occur as an endpoint of chronic obstructive atherosclerosis, persistant infections by intracellular bacteria or parasitic protozoae, non-malignant hyperproliferative disorders, malignant hyperproliferative disorders, liver steatosis and associated syndromes and cholestatic and fibrotic effects that are associated with alcohol-induced cirrhosis or with viral-borne forms of hepatitis.

### Brief description of the drawings

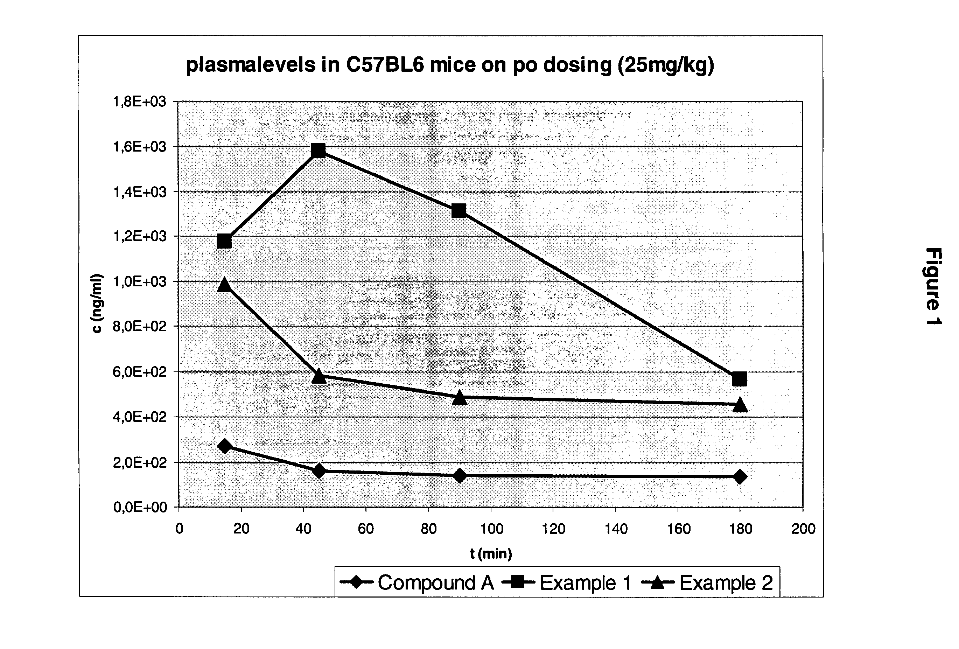

Figure 1 shows the plasma concentration in C57BL6 mice on perorally application.

The compounds of the present invention are defined by formula (1): 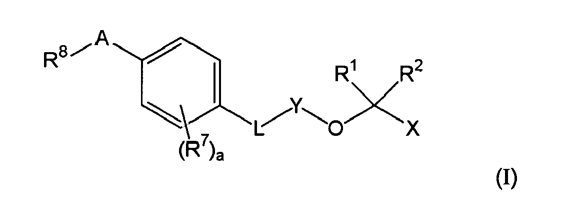 or an enantiomer, diastereomer, tautomer, solvate or pharmaceutically acceptable salt thereof,
wherein
- R¹ and R²: are independently from each other selected from hydrogen, fluorine and C₁-C₃ alkyl;
- X is:

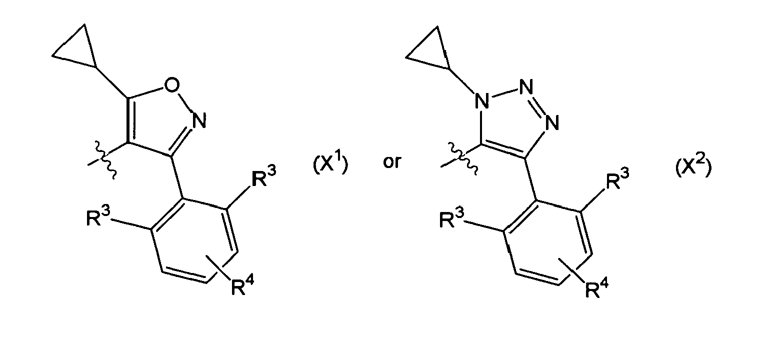

- R³ is: independently selected from hydrogen, halogen, C₁-C₃ alkyl and O-C₁-C₃ alkyl wherein each alkyl is unsubstituted or substituted with one or more fluorine atoms;
- R⁴ is: hydrogen or fluorine;
- Y is: selected from

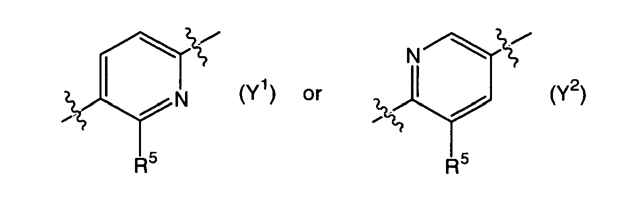

- R⁵ is: halogen, cyano, C₁-C₃ alkyl, C₂-C₃ alkenyl, O-C₁-C₃ alkyl or C₃-C₆ cycloalkyl, wherein each alkyl, alkenyl and cycloalkyl is unsubstituted or substituted with one to five substituents R¹⁰;
- L is: -CH₂-N(R⁶)-, -CH₂O- or O-CH₂;
- R⁶ is: C₁-C₃-alkyl, or C₃-C₅ cycloalkyl, wherein each alkyl or cycloalkyl is unsubstituted or substituted with 1-5 fluorine atoms;
- R⁷ is: halogen, C₁-C₆ alkyl, C₃-C₆ cycloalkyl, C₂-C₆ alkenyl, C₂-C₆ alkynyl, cyano, O-C₁-C₆ alkyl or O-C₃-C₆ cycloalkyl, wherein each alkyl, alkenyl, alkynyl and cycloalkyl is unsubstituted or substituted with one to five substituents R¹⁰;
- A is: a bond, CH₂, CHCH₃, C(CH₃)₂, -CH₂O- -CH₂CH₂- or -CH=CH-;
- R⁸ is: COOR⁹, CONHR⁹, C(O)NHSO₂R⁹ or tetrazole which is connected to A via the C-atom;
- R⁹ is: hydrogen, C₁-C₆-alkyl, C₃-C₆-cycloalkyl or a 4-7-membered heterocyclic ring, wherein the alkyl, cycloalkyl and heterocyclyl groups are unsubstituted or substituted with one to three substituents R¹⁰;
- R¹⁰ is: independently selected from hydrogen, halogen, cyano, nitro, azido, =O =S, C₁-C₆ alkyl, C₂-C₆ alkenyl, C₂-C₆ alkynyl, C₃-C₆ cycloalkyl, 4-7 membered heterocyclyl, aryl, heteroaryl, NR¹¹R¹² NR¹¹SO₂R¹² NR¹¹C(O)OR¹² NR¹¹C(O)R¹², -OR¹¹, OC(O)R¹¹, SO₂R¹¹, SO₃R¹¹, SO₂NR¹¹R¹², C(O)R¹¹, C(O)OR¹¹ and C(O)NR¹¹R¹²;
- R¹¹ and R¹²: are independently from each other selected from hydrogen, C₁-C₆ alkyl and C₃-C₆ cycloalkyl, wherein each alkyl or cycloalkyl may be unsubstituted or substituted with one to five fluorines and/or one to three substituents selected from OH, OCH₃, OCH₂F, OCHF₂, OCF₃, =O NH₂, NHCH₃ and N(CH₃)₂; and
- a is: 0, 1, 2 or 3.

R¹ and R² are independently from each other defined as above. Preferably, R¹ and R² simultaneously represent a hydrogen atom.

It is preferred that X is defined by the group (X¹) wherein R³ and R⁴ are defined as above.

R³ is defined as above. In a preferred embodiment, R³ is independently selected from the group consisting of hydrogen, fluorine, chlorine, CF₃ and OCF₃. More preferably, both substituents R³ represent a chlorine atom.

The radical R⁴ may be located on the meta or para position of the phenyl ring with reference to the isoxazole ring. Preferably, R⁴ is located on the para position of the phenyl ring. Preferably, R⁴ is a hydrogen atom.
Y is preferably (Y¹) wherein R⁵ is defined as above.
Preferably, R⁵ is a CH₃ or CF₃ group, more preferably, R⁵ is a CF₃ group.

L is defined as above. In a preferred embodiment, L is -CH₂N(R⁶)-. Therein, R⁶ is a C₁-C₃ alkyl or C₃-C₅ cycloalkyl group wherein alkyl or cycloalkyl is unsubstituted or substituted with 1 to 5 fluorine atoms. Preferably, R⁶ is a C₁-C₃ alkyl group, more preferably, R⁶ is a methyl group.

R⁷ is defined as above. In a preferred embodiment, R⁷ is selected from C₁-C₆ alkyl and O-C₁-C₆ alkyl.

The index a represents an integer selected from 0, 1, 2, or 3. Preferably, a is 0 or 1, more preferably, a is 0.

The variant A preferably represents a bond.

It is further preferred that the variant R⁸ is COOR⁹ wherein R⁹ is defined as above. Preferably, R⁹ is hydrogen or C₁-C₆ alkyl, more preferably, R⁹ is hydrogen.

Preferred compounds of formula (I) are those compounds in which one or more of the residues contained therein have the meanings given above. It is understood, that the claimed compounds cover any compound obtained by combining any of the definitions disclosed within this description for the various substituents. With respect to all compounds of formula (I), the present invention also includes all tautomeric and stereoisomeric forms, solvates and mixtures thereof in all ratios, and their pharmaceutically acceptable salts.

In the above and the following, the employed terms have independently the meaning as described below:

Aryl is an aromatic mono- or polycyclic moiety with preferably 6 to 20 carbon atoms, more preferably 6, 7, 8, 9 or 10 carbon atoms, which moiety is preferably selected from phenyl, biphenyl, naphthyl, tetrahydronaphthyl, fluorenyl, indenyl and phenanthrenyl, more preferably phenyl and naphthyl.

Heteroaryl is a monocyclic or polycyclic aromatic moiety having 5 to 20 carbon atoms with at least one ring containing a heteroatom selected from O, N and/or S, or heteroaryl is an aromatic ring containing at least one heteroatom selected from O, N and/or S and 1, 2, 3, 4, 5 or 6 carbon atoms. Preferably, heteroaryl contains 1, 2, 3 or 4, more preferably 1, 2 or 3 heteroatoms selected from O and/or N and is preferably selected from pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, triazolyl, thiadiazolyl, thiadiazolyl, furazanyl, benzofurazanyl, benzothiophenyl, benzothiazolyl, benzoxazolyl, quinazolinyl, quinoxalinyl, naphthyridinyl and furopyridinyl. Spiro moieties are also included within the scope of this definition. Preferred heteroaryl includes pyridinyl, imidazolyl, pyrimidinyl, pyrazolyl, triazolyl, pyrazinyl, tetrazolyl, isoxazolyl, oxazolyl, isothiazolyl, oxadiazolyl and triazolyl.

Heterocyclyl is a 3 to 10-membered saturated or unsaturated ring containing at least one heteroatom selected from O, N and/or S and 1, 2, 3, 4, 5 or 6 carbon atoms. Preferably, heterocyclyl contains 1, 2, 3 or 4, more preferably 1, 2 or 3 heteroatoms selected from O and/or N. Heterocyclyl includes mono- and bicyclic ringsystems and is preferably selected from pyrrolidinyl, tetrahydrofuranyl, dihydrofuranyl, tetrahydrothienyl, tetrahydropyranyl, dihydropyranyl, tetrahydrothiopyranyl, piperidino, morpholino, thiomorpholino, thioxanyl, piperazinyl, homopiperazinyl, azetidinyl, oxetanyl, thietanyl, homopiperidinyl, oxepanyl, thiepanyl, oxazepinyl, diazepinyl, thiazepinyl, 1,2,3,6-tetrahydropyridinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinylimidazolinyl, imidazolidinyl, azetidin-2-one-1-yl, pyrrolidin-2-one-1-yl, piperid-2-one-1-yl, azepan-2-one-1-yl, 3-azabicyco[3.1.0] hexanyl, 3-azabicyclo[4.1.0]heptanyl, azabicyclo[2.2.2]hexanyl, 3H-indolyl and quinolizinyl. Spiromoieties are also included within the scope of this definition.

C₁-C₆ Alkyl is a saturated hydrocarbon moiety, namely straight chain or branched alkyl having 1, 2, 3, 4, 5 or 6 carbon atoms, preferably 1, 2 or 3 carbon atoms, such as methyl, ethyl, n-propyl, isopropyl, n-butyl, isobutyl, tert-butyl, n-pentyl, isopentyl, neopentyl or hexyl.

C₃-C₆ Cycloalkyl is an alkyl ring having 3, 4, 5 or 6 carbons, such as cyclopropyl, cyclobutyl, cyclopentyl or cyclohexyl.

Carbocyclyl is a monocyclic or polycyclic ring system of 3 to 20 carbon atoms which may be saturated or unsaturated. Thus, the term "carbocyclyl" includes cycloalkyls as defined above as well as partially unsaturated carbocyclic groups such as cyclopentene, cyclopentadiene or cyclohexene.

C₂-C₆ Alkenyl is an unsaturated hydrocarbon moiety with one or more double bonds, preferably one double bond, namely straight chain or branched alkenyl having 2, 3, 4, 5 or 6 carbon atoms, such as vinyl, allyl, methallyl, buten-2-yl, buten-3-yl, penten-2-yl, penten-3-yl, penten-4-yl, 3-methyl-but-3-enyl, 2-methyl-but-3-enyl, 1-methyl-but-3-enyl or hexenyl.

C₂-C₆ Alkynyl is an unsaturated hydrocarbon moiety with one or more triple bonds, preferably one triple bond, namely straight chain or branched alkynyl having 2, 3, 4, 5 or 6 carbon atoms, such as ethynyl, propynyl, butyn-2-yl, butyn-3-yl, pentyn-2-yl, pentyn-3-yl, pentyn-4-yl, 2-methyl-but-3-ynyl, 1-methyl-but-3-ynyl or hexynyl.

Halo or halogen is a halogen atom selected from F, Cl, Br and I, preferably F, Cl and Br.

Preferred embodiments of the compounds according to the present invention are shown below.

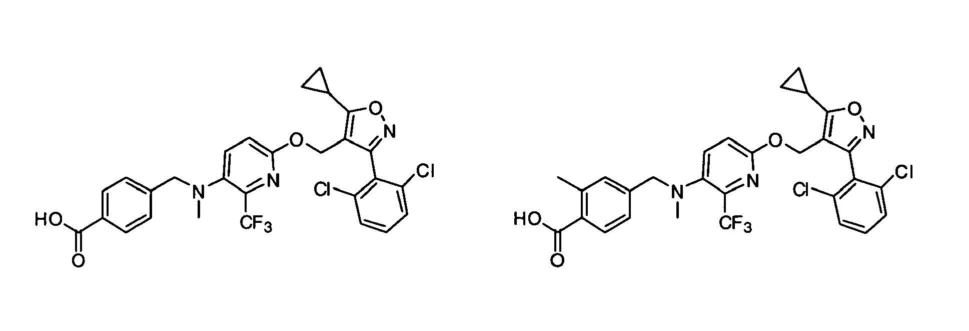

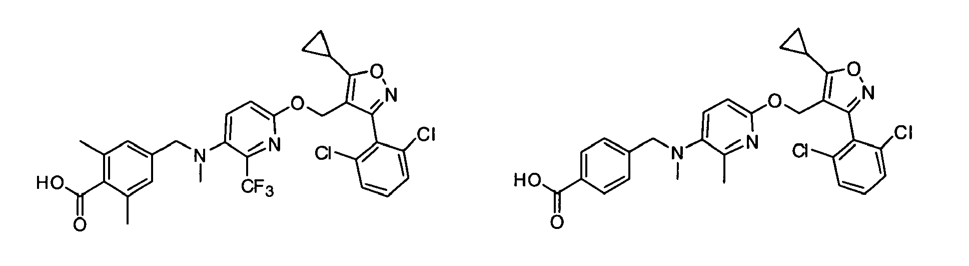

The compounds of the present invention can be in the form of a prodrug compound. "Prodrug compound" means a derivative that is converted into a compound according to the present invention by a reaction with an enzyme, gastric acid or the like under a physiological condition in the living body, e.g. by oxidation, reduction, hydrolysis or the like, each of which is carried out enzymatically. Examples of the prodrug are compounds, wherein the amino group in a compound of the present invention is acylated, alkylated or phosphorylated to form, e.g., eicosanoylamino, alanylamino, pivaloyloxymethylamino or wherein the hydroxyl group is acylated, alkylated, phosphorylated or converted into the borate, e.g. acetyloxy, palmitoyloxy, pivaloyloxy, succinyloxy, fumaryloxy, alanyloxy or wherein the carboxyl group is esterified or amidated. These compounds can be produced from compounds of the present invention according to well-known methods. Other examples of the prodrug are compounds, wherein the carboxylate in a compound of the present invention is, for example, converted into an alkyl-, aryl-, choline-, amino, acyloxymethylester, linolenoyl-ester.

Metabolites of compounds of the present invention are also within the scope of the present invention.

Where tautomerism, like e.g. keto-enol tautomerism, of compounds of the present invention or their prodrugs may occur, the individual forms, like e.g. the keto and enol form, are each within the scope of the invention as well as their mixtures in any ratio. Same applies for stereoisomers, like e.g. enantiomers, cis/trans isomers, conformers and the like.

If desired, isomers can be separated by methods well known in the art, e.g. by liquid chromatography. Same applies for enantiomers by using e.g. chiral stationary phases. Additionally, enantiomers may be isolated by converting them into diastereomers, i.e. coupling with an enantiomerically pure auxiliary compound, subsequent separation of the resulting diastereomers and cleavage of the auxiliary residue. Alternatively, any enantiomer of a compound of the present invention may be obtained from stereoselective synthesis using optically pure starting materials.

The compounds of the present invention can be in the form of a pharmaceutically acceptable salt or a solvate. The term "pharmaceutically acceptable salts" refers to salts prepared from pharmaceutically acceptable non-toxic bases or acids, including inorganic bases or acids and organic bases or acids. In case the compounds of the present invention contain one or more acidic or basic groups, the invention also comprises their corresponding pharmaceutically or toxicologically acceptable salts, in particular their pharmaceutically utilizable salts. Thus, the compounds of the present invention which contain acidic groups can be present on these groups and can be used according to the invention, for example, as alkali metal salts, alkaline earth metal salts or ammonium salts. More precise examples of such salts include sodium salts, potassium salts, calcium salts, magnesium salts or salts with ammonia or organic amines such as, for example, ethylamine, ethanolamine, triethanolamine or amino acids. The compounds of the present invention which contain one or more basic groups, i.e. groups which can be protonated, can be present and can be used according to the invention in the form of their addition salts with inorganic or organic acids. Examples of suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the present invention simultaneously contain acidic and basic groups in the molecule, the invention also includes, in addition to the salt forms mentioned, inner salts or betaines (zwitterions). The respective salts can be obtained by customary methods which are known to the person skilled in the art like, for example, by contacting these with an organic or inorganic acid or base in a solvent or dispersant, or by anion exchange or cation exchange with other salts. The present invention also includes all salts of the compounds of the present invention which, owing to low physiological compatibility, are not directly suitable for use in pharmaceuticals but which can be used, for example, as intermediates for chemical reactions or for the preparation of pharmaceutically acceptable salts.

Furthermore, the present invention provides pharmaceutical compositions comprising at least one compound of the present invention, or a prodrug compound thereof, or a pharmaceutically acceptable salt or solvate thereof as active ingredient together with a pharmaceutically acceptable carrier.

"Pharmaceutical composition" means one or more active ingredients, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing at least one compound of the present invention and a pharmaceutically acceptable carrier.

The pharmaceutical composition of the present invention may additionally comprise one or more other compounds as active ingredients like a prodrug compound or other nuclear receptor modulators.

The compositions are suitable for oral, rectal, topical, parenteral (including subcutaneous, intramuscular, and intravenous), ocular (ophthalmic), pulmonary (nasal or buccal inhalation) or nasal administration, although the most suitable route in any given case will depend on the nature and severity of the conditions being treated and on the nature of the active ingredient. They may be conveniently presented in unit dosage form and prepared by any of the methods well-known in the art of pharmacy.

The compounds of the present invention bind to the NR1H4 receptor (FXR) and act as agonists or partial agonists of the NR1H4 receptor (FXR).

FXR is proposed to be a nuclear bile acid sensor. As a result, it modulates both, the synthetic output of bile acids in the liver and their recycling in the intestine (by regulating bile acid binding proteins). But beyond bile acid physiology, FXR seems to be involved in the regulation of many diverse physiological processes which are relevant in the etiology and for the treatment of diseases as diverse as cholesterol gallstones, metabolic disorders such as Type II Diabetes, dyslipidemias or obesity, chronic inflammatory diseases such as Inflammatory Bowel Diseases or chronic intrahepatic forms of cholestasis and many others diseases (T. Claudel et al. "The Farnesoid X receptor: a molecular link between bile acid and lipid and glucose metabolism" Arterioscler. Thromb. Vasc. Biol. 2005, 25(10), 2020-2030; S. Westin et al. "FXR, a therapeutic target for bile acid and lipid disorders" Mini Rev. Med. Chem. 2005, 5(8), 719-727).

FXR regulates a complex pattern of response genes in the liver. The gene products have impact on diverse physiological processes. In the course of functional analysis of FXR, the first regulatory network that was analyzed was the regulation of bile acid synthesis. While the LXRs induce the key enzyme of the conversion of cholesterol into bile acids, Cyp7A1, via the induction of the regulatory nuclear receptor LRH-1, FXR represses the induction of Cyp7A1 via the upregulation of mRNA encoding SHP, a further nuclear receptor that is dominant repressive over LRH-1. Since FXR binds the end products of this pathway, primary bile acids such as cholic acid (CA) or chenodeoxycholic acid (CDCA), this can be regarded as an example of feedback inhibition on the gene expression level (B. Goodwin et al. "A regulatory cascade of the nuclear receptors FXR, SHP-1, and LRH-1 represses bile acid biosynthesis" Mol. Cell 2000, 6(3), 517-526; T. Lu et al. "Molecular basis for feedback regulation of bile acid synthesis by nuclear receptors" Mol. Cell 2000, 6(3), 507-515). Parallel to the repression of bile acid synthesis via SHP, FXR induces a range of so-called ABC (for ATP-binding cassette) transporters that are responsible for the export of toxic bile acids from the hepatocyte cytosol into the canaliculi, the small bile duct ramifications where the bile originates. This hepatoprotective function of FXR became first apparent with the analysis of FXR knockout mice (C. Sinal et al. "Targeted disruption of the nuclear receptor FXR/BAR impairs bile acid and lipid homeostasis" Cell 2000, 102(6), 731-744). where under- or overexpression of several ABC-transporters in the liver was shown. Further detailed analysis revealed that the major bile salt excretory pump BSEP or ABCB11 (M. Ananthanarayananet al. "Human bile salt export pump promoter is transactivated by the farnesoid X receptor/bile acid receptor" J. Biol. Chem. 2001, 276(31), 28857-28865; J. Plass et al. "Farnesoid X receptor and bile salts are involved in transcriptional regulation of the gene encoding the human bile salt export pump" Hepatology 2002, 35(3), 589-96) as well as the key enzyme which mediates lipid transfer from lipoproteins to phospholipids, PLTP (N. Urizar et al. "The farnesoid X-activated receptor mediates bile acid activation of phospholipid transfer protein gene expression" J. Biol. Chem. 2000, 275(50), 39313-39317), and the two key canalicular membrane transporters for phospholipids, MRP-2 (ABCC4) (H. Kast et al. "Regulation of multidrug resistance-associated protein 2 (ABCC2) by the nuclear receptors pregnane X receptor, farnesoid X-activated receptor, and constitutive androstane receptor" J. Biol. Chem. 2002, 277(4), 2908-2915) and MDR-3 (ABCB4); L. Huang et al. "Farnesoid X receptor activates transcription of the phospholipid pump MDR3" J. Biol. Chem. 2003, 278(51), 51085-51090) are direct targets for ligand-directed transcriptional activation by FXR (summarized in: M. Miyata "Role of farnesoid X receptor in the enhancement of canalicular bile acid output and excretion of unconjugated bile acids: a mechanism for protection against cholic acid-induced liver toxicity", J. Pharmacol. Exp. Ther. 2005, 312(2), 759-766; G. Rizzo et al. "Role of FXR in regulating bile acid homeostasis and relevance for human diseases" Curr. Drug Targets Immune Endocr. Metabol. Disord. 2005, 5(3), 289-303.)

The fact that FXR seems to be the major metabolite sensor and regulator for the synthesis, export and re-circulation of bile acids suggested the use of FXR ligands to induce bile flow and change bile acid composition towards more hydrophilic composition. With the development of the first synthetic FXR ligand GW4064 (P. Maloney et al. "Identification of a chemical tool for the orphan nuclear receptor FXR" J. Med. Chem. 2000, 43(16), 2971-2974; T. Willson et al. "Chemical genomics: functional analysis of orphan nuclear receptors in the regulation of bile acid metabolism" Med. Res. Rev. 2001, 21(6) 513-22) as a tool compound and of the semi-synthetic artificial bile acid ligand 6-alpha-ethyl-CDCA, the effects of superstimulation of FXR by potent agonists could be analyzed. It was shown that both ligands induce bile flow in bile duct ligated animals. Moreover, in addition to choleretic effects, also hepatoprotective effects could be demonstrated (R. Pellicciari et al. "6alpha-ethyl-chenodeoxycholic acid (6-ECDCA), a potent and selective FXR agonist endowed with anticholestatic activity" J. Med. Chem. 2002, 45(17), 3569-3572; Y. Liu et al. "Hepatoprotection by the farnesoid X receptor agonist GW4064 in rat models of intra- and extrahepatic cholestasis" J. Clin. Invest. 2003, 112(11), 1678-1687). This hepatoprotective effect was further narrowed down to an anti-fibrotic effect that results from the repression of Tissue Inhibitors of Matrix-Metalloproteinases, TIMP-1 and 2, the induction of collagen-deposit resolving Matrix-Metalloproteinase 2 (MMP-2) in hepatic stellate cells and the subsequent reduction of alpha-collagen mRNA and Transforming growth factor beta (TGF-beta) mRNA which are both pro-fibrotic factors by FXR agonists (S. Fiorucci et al. "The nuclear receptor SHP mediates inhibition of hepatic stellate cells by FXR and protects against liver fibrosis", Gastroenterology 2004, 127(5), 1497-1512; S. Fiorucci et al. "A farnesoid x receptor-small heterodimer partner regulatory cascade modulates tissue metalloproteinase inhibitor-1 and matrix metalloprotease expression in hepatic stellate cells and promotes resolution of liver fibrosis"J. Pharmacol. Exp. Ther. 2005, 314(2), 584-595). The anti-fibrotic activity of FXR is at least partially mediated by the induction of PPARgamma, a further nuclear receptor, with which anti-fibrotic activity is associated (S. Fiorucci et al. "Cross-talk between farnesoid-X-receptor (FXR) and peroxisome proliferator-activated receptor gamma contributes to the antifibrotic activity of FXR ligands in rodent models of liver cirrhosis" J. Pharmacol. Exp. Ther. 2005, 315(1), 58-68; A. Galli et al. "Antidiabetic thiazolidinediones inhibit collagen synthesis and hepatic stellate cell activation in vivo and in vitro" Gastroenterology 2002, 122(7), 1924-1940; I. Pineda Torra et al., "Bile acids induce the expression of the human peroxisome proliferator-activated receptor alpha gene via activation of the farnesoid X receptor" Mol. Endocrinol. 2003, 17(2), 259-272). Furthermore, anti-cholestatic activity was demonstrated in bile-duct ligated animal models as well as in animal models of estrogen-induced cholestasis (S. Fiorucci et al. "Protective effects of 6-ethyl chenodeoxycholic acid, a farnesoid X receptor ligand, in estrogen-induced cholestasis" J. Pharmacol. Exp. Ther. 2005, 313(2), 604-612).

Genetic studies demonstrate that in hereditary forms of cholestasis (Progressive Familiar Intrahepatic Cholestasis = PFIC, Type I - IV) either nuclear localization of FXR itself is reduced as a consequence of a mutation in the FIC1 gene (in PFIC Type I, also called Byler's Disease) (F. Chen et al. "Progressive familial intrahepatic cholestasis, type 1, is associated with decreased farnesoid X receptor activity" Gastroenterology. 2004, 126(3), 756-64; L. Alvarez et al. "Reduced hepatic expression of farnesoid X receptor in hereditary cholestasis associated to mutation in ATP8B1" Hum. Mol. Genet. 2004; 13(20), 2451-60) or levels of the FXR target gene encoding MDR-3 phospholipid export pump are reduced (in PFIC Type III). Taken together there is a growing body of evidence that FXR binding compounds will demonstrate substantial clinical utility in the therapeutic regimen of chronic cholestatic conditions such as Primary Biliary Cirrhosis (PBC) or Primary Sclerosing Cholangitis (PSC) (reviewed in: G. Rizzo et al. Curr. Drug Targets Immune Endocr. Metabol. Disord. 2005, 5(3), 289-303; G. Zollner "Role of nuclear receptors in the adaptive response to bile acids and cholestasis: pathogenetic and therapeutic considerations" Mol. Pharm. 2006, 3(3), 231-51, S. Cai et al. "FXR: a target for cholestatic syndromes?" Expert Opin. Ther. Targets 2006, 10(3), 409-421).

The deep impact that FXR activation has on bile acid metabolism and excretion is not only relevant for cholestatic syndromes but even more directly for a therapy against gallstone formation. Cholesterol gallstones form due to low solubility of cholesterol that is actively pumped out of the liver cell into the lumen of the canaliculi. It is the relative percentage of content of the three major components, bile acids, phospholipids and free cholesterol that determines the formation of mixed micelles and hence apparent solubility of free cholesterol in the bile. FXR polymorphisms map as quantitative trait loci as one factor contributing to gallstone disease (H. Wittenburg "FXR and ABCG5/ABCG8 as determinants of cholesterol gallstone formation from quantitative trait locus mapping in mice", Gastroenterology 2003, 125(3), 868-881). Using the synthetic FXR tool compound GW4064 it could be demonstrated that activation of FXR leads to an improvement of the Cholesterol Saturation Index (=CSI) and directly to an abolishment of gallstone formation in C57L gallstone susceptible mice whereas drug treatment in FXR knockout mice shows no effect on gallstone formation (A. Moschetta et al. "Prevention of cholesterol gallstone disease by FXR agonists in a mouse model" Nature Medicine 2004, 10(12), 1352-1358).

These results qualify FXR as a good target for the development of small molecule agonists that can be used to prevent cholesterol gallstone formation or to prevent reformation of gallstones after surgical removal or shockwave lithotripsy (discussed in:
S. Doggrell "New targets in and potential treatments for cholesterol gallstone disease" Curr. Opin. Investig. Drugs 2006, 7(4), 344-348).

Thus, in one embodiment of the invention, the compound according to formula (I) and pharmaceutical compositions comprising said compound is used for the prophylaxis and/or treatment of obstructive or chronic inflammatory disorders that arise out of improper bile composition such as cholelithiasis also known as cholesterol gallstones.

Since the discovery of the first synthetic FXR agonist and its administration to rodents it became evident that FXR is a key regulator of serum triglycerides (P. Maloney et al. J. Med. Chem. 2000, 43(16), 2971-2974; T. Willson et al. Med. Res. Rev. 2001, 21(6), 513-22). Over the past six years accumulating evidence has been published that activation of FXR by synthetic agonists leads to significant reduction of serum triglycerides, mainly in the form of reduced VLDL, but also to reduced total serum cholesterol (H. Kast et al. "Farnesoid X-activated receptor induces apolipoprotein C-II transcription: a molecular mechanism linking plasma triglyceride levels to bile acids" Mol. Endocrinol. 2001, 15(10), 1720-1728; N.Urizar et al. "A natural product that lowers cholesterol as an antagonist ligand for FXR" Science 2002, 296(5573), 1703-1706; G. Lambert et al. "The farnesoid X-receptor is an essential regulator of cholesterol homeostasis" J. Biol. Chem. 2003, 278, 2563-2570; M. Watanabe et al. "Bile acids lower triglyceride levels via a pathway involving FXR, SHP, and SREBP-1c" J. Clin. Invest. 2004, 113(10), 1408-1418; A. Figge et al. "Hepatic overexpression of murine Abcb11 increases hepatobiliary lipid secretion and reduces hepatic steatosis "J. Biol. Chem. 2004, 279(4), 2790-2799; S. Bilz et al. "Activation of the farnesoid X receptor improves lipid metabolism in combined hyperlipidemic hamsters" Am. J. Physiol. Endocrinol. Metab. 2006, 290(4), E716-22).

But the lowering of serum triglycerides is not a stand alone effect. Treatment of db/db or ob/ob mice with synthetic FXR agonist GW4064 resulted in marked and combined reduction of serum triglycerides, total cholesterol, free fatty acids, ketone bodies such as 3-OH Butyrate. Moreover, FXR activation engages with the intracellular insulin signaling pathway in hepatocytes, resulting in reduced output of glucose from liver gluconeogenesis but concomitant increase in liver glycogen. Insulin sensitivity as well as glucose tolerance were positively impacted by FXR treatment (K. Stayrook et al. "Regulation of carbohydrate metabolism by the farnesoid X receptor" Endocrinology 2005, 146(3), 984-91; Y. Zhang et al. "Activation of the nuclear receptor FXR improves hyperglycemia and hyperlipidemia in diabetic mice" Proc. Natl. Acad. Sci. USA 2006, 103(4), 1006-1011; B. Cariou et al. "The farnesoid X receptor modulates adiposity and peripheral insulin sensitivity in mice" J. Biol. Chem. 2006, 281, 11039-11049; K. Ma et al. "Farnesoid X receptor is essential for normal glucose homeostasis" J. Clin. Invest. 2006, 116(4), 1102-1109; D. Duran-Sandoval et al. "Potential regulatory role of the farnesoid X receptor in the metabolic syndrome" Biochimie 2005, 87(1), 93-98). An effect on reduction of body weight was also recently observed in mice overfed with a high lipid diet (C. Lihong et al."FXR Agonist, GW4064, Reverses Metabolic Defects in High-Fat Diet Fed Mice" American Diabetes Association (ADA) 66th annual scientific sessions, June 2006, Abstract Number 856-P). This weight loss effect might results from FXR's induction of FGF-19, a fibroblast growth factor that is known to lead to weight loss and athletic phenotype (J. Holt et al. Genes Dev. 2003, 17(13), 1581-1591; E. Tomlinson et al. "Transgenic mice expressing human fibroblast growth factor-19 display increased metabolic rate and decreased adiposity" Endocrinology 2002, 143(5), 1741-1747). In recent patent applications, the effect of FXR agonist on reduction of body weight was demonstrated (Stoffel W. et al. "Methods for inhibiting Adipogenesis and for treating Type 2 Diabetes" International Patent Application WO 2004/087076; S. Jones et al "Methods of using FXR Agonists" International Patent Application WO 2003/080803).

Taken together, these pharmacological effects of FXR agonists can be exploited in different therapeutic ways: FXR binding compounds are thought to be good candidates for the treatment of Type II Diabetes because of their insulin sensitization, glycogenogenic, and lipid lowering effects.

In one embodiment, the compounds according to the invention and pharmaceutical compositions comprising said compounds are used in the prophylaxis and/or treatment of Type II Diabetes which can be overcome by FXR-mediated upregulation of systemic insulin sensitivity and intracellular insulin signalling in liver, increased peripheral glucose uptake and metabolisation, increased glycogen storage in liver, decreased output of glucose into serum from liver-borne gluconeogenesis.

In a further embodiment, said compounds and pharmaceutical compositions are used for the prophylaxis and/or treatment of chronic intrahepatic, such as primary biliary cirrhosis (PBC), primary sclerosing cholangitis (PSC), progressive familiar cholestasis (PFIC), alcohol-induced cirrhosis and associated cholestasis, and some forms of extrahepatic cholestatic conditions, or liver fibrosis resulting from chronic cholestatic conditions or acute intraheptic cholestatic conditions such as estrogen or drug induced cholestasis.

The invention also relates to a compound of formula (I) or to a pharmaceutical composition comprising said compound for the prophylaxis and/or treatment of gastrointestinal conditions with a reduced uptake of dietary fat and fat-soluble dietary vitamins which can be overcome by increased intestinal levels of bile acids and phospholipids.

In a further embodiment, said compound or pharmaceutical composition is used for preventing and/or treating a disease selected from the group consisting of lipid and lipoprotein disorders such as hypercholesterolemia, hypertriglyceridemia, and atherosclerosis as a clinically manifest condition which can be ameliorated by FXR's beneficial effect on raising HDL cholesterol, lowering serum triglycerides, increasing conversion of liver cholesterol into bile acids and increased clearance and metabolic conversion of VLDL and other lipoproteins in the liver.

In one further embodiment, said compound and pharmaceutical composition are used for the prophylaxis and/or treatment of diseases where the combined lipid lowering, anti-cholestatic and anti-fibrotic effects of FXR-targeted medicaments can be exploited for the treatment of liver steatosis and associated syndromes such as non-alcoholic steatohepatitis ("NASH"), or for the treatment of cholestatic and fibrotic effects that are associated with alcohol-induced cirrhosis, or with viral-borne forms of hepatitis.

In conjunction with the hypolipidemic effects it was also shown that loss of functional FXR leads to increased atherosclerosis in ApoE knockout mice (E. Hanniman et al. "Loss of functional farnesoid X receptor increases atherosclerotic lesions in apolipoprotein E-deficient mice" J. Lipid Res. 2005, 46(12), 2595-2604). Therefore, FXR agonists might have clinical utility as anti-atherosclerotic and cardioprotective drugs. The downregulation of Endothelin-1 in Vascular Smooth Muscle Cells might also contribute to such beneficial therapeutic effects (F. He et al. "Downregulation of endothelin-1 by farnesoid X receptor in vascular endothelial cells" Circ. Res. 2006, 98(2), 192-9).

The invention also relates to a compound according to formula (I) or a pharmaceutical composition comprising said compound for preventive and posttraumatic treatment of cardiovascular disorders such as acute myocardial infarction, acute stroke, or thrombosis which occur as an endpoint of chronic obstructive atherosclerosis.

In a few selected publications the effects of FXR and FXR agonists on proliferation of cancer and non-malignant cells and apoptosis have been assessed. From these preliminary results it seems as if FXR agonists might also influence apoptosis in cancer cell lines (E. Niesor et al. "The nuclear receptors FXR and LXRalpha: potential targets for the development of drugs affecting lipid metabolism and neoplastic diseases" Curr. Pharm. Des. 2001, 7(4), 231-59) and in Vascular Smooth Muscle Cells (VSMCs) (D. Bishop-Bailey et al. "Expression and activation of the farnesoid X receptor in the vasculature" Proc. Natl. Acad. Sci. U S A. 2004, 101(10), 3668-3673). Furthermore, FXR seems to be expressed in metastasizing breast cancer cells and in colon cancer (J. Silva "Lipids isolated from bone induce the migration of human breast cancer cells" J. Lipid Res. 2006, 47(4), 724-733; G. De Gottardi et al. "The bile acid nuclear receptor FXR and the bile acid binding protein IBABP are differently expressed in colon cancer" Dig. Dis. Sci. 2004, 49(6), 982-989). Other publications that focus primarily on FXR's effect on metabolism draw a line to intracellular signaling from FXR via the Forkhead /Wingless (FOXO) family of transcriptional modulators to the Phosphatidylinositol-trisphosphat (P1₃)- Kinase / Akt signal transduction pathway (D. Duran-Sandoval et al. J. Biol. Chem. 2005, 280(33), 29971-29979; Y. Zhang et al. Proc. Natl. Acad. Sci. U S A. 2006, 103(4), 1006-1011) that is similarily employed by insulin intracellular signaling as well as neoplastically transformed cells.

This would allow to regard FXR also as a potential target for the treatment of proliferative diseases, especially metastasizing cancer forms that overexpress FXR or those where the FOXO /PI₃- Kinase / Akt Pathway is responsible for driving proliferation.

Therefore, the compounds according to formula (I) or pharmaceutical composition comprising said compounds are suitable for preventing and/or treating non-malignant hyperproliferative disorders such as increased neointima formation after balloon vessel dilatation and stent application due to increased proliferation of vascular smooth muscle cells (VSMCs) or Bening Prostate Hyperplasia (BPH), a pre-neoplastic form of hyperproliferation, other forms of scar tissue formation and fibrotisation which can be overcome by e.g. FXR-mediated intervention into the PI-3Kinase / AKT / mTOR intracellular signalling pathway, reduction in Matrix-Metalloproteinase activity and alpha-Collagen deposition.

In a further embodiment, said compounds and pharmaceutical compositions are used for the prophylaxis and/or treatment of malignant hyperproliferative disorders such as all forms of cancer (e.g. certain forms of breast, prostate cancer or colon cancer) where interference with PI-3-Kinase/AKT/mTOR signalling and / or induction of p27^{kip} and / or induction of apoptosis will have a beneficial impact.

Finally, FXR seems also to be involved in the control of antibacterial defense in the intestine (T. Inagaki et al. "Regulation of antibacterial defense in the small intestine by the nuclear bile acid receptor" Proc. Natl. Acad. Sci. U S A. 2006, 103(10), 3920-3905) although an exact mechanism is not provided. From these published data, however, one can conclude that treatment with FXR agonists might have a beneficial impact in the therapy of Inflammatory Bowel Disorders (IBD), in particular those forms where the upper (ileal) part of the intestine is affected (e.g. ileal Crohn's disease) because this seems to be the site of action of FXR's control on bacterial growth. In IBD the desensitization of the adaptive immune response is somehow impaired in the intestinal immune system. Bacterial overgrowth might then be the causative trigger towards establishment of a chronic inflammatory response. Hence, dampening of bacterial growth by FXR-borne mechanisms might be a key mechanism to prevent acute inflammatory episodes.

Thus, the invention also relates to a compound according to formula (I) or a pharmaceutical composition comprising said compound for preventing and/or treating a disease related to Inflammatory Bowel Diseases such as Crohn's disease or Colitis ulcerosa. FXR-mediated restoration of intestinal barrier function and reduction in non-commensal bacterial load is believed to be helpful in reducing the exposure of bacterial antigens to the intestinal immune system and can therefore reduce inflammatory responses.

The invention further relates to a compound or pharmaceutical composition for the prophylaxis and/or treatment of obesity and associated disorders such as metabolic syndrome (combined conditions of dyslipidemias, diabetes and abnormally high body-mass index) which can be overcome by FXR-mediated lowering of serum triglycerides, blood glucose and increased insulin sensitivity and FXR-mediated weight loss.

In one embodiment, said compound or pharmaceutical composition is for treating persistent infections by intracellular bacteria or parasitic protozoae such as *Mycobacterium spec*. (Treatment of Tuberculosis or Lepra), *Listeria monocytogenes* (Treatment of Listeriosis), *Leishmania spec*. (Leishmaniosis), *Trypanosoma spec*. (Chagas Disease; Trypanosomiasis; Sleeping Sickness).

In a further embodiment, the compounds or pharmaceutical composition of the present invention are useful in preventing and/or treating clinical complications of Type I and Type II Diabetes. Examples of such complications include Diabetic Nephropathy, Diabetic Retinopathy, Diabetic Neuropathies, Peripheral Arterial Occlusive Disease (PAOD). Other clinical complications of Diabetes are also encompassed by the present invention.

Furthermore, conditions and diseases which result from chronic fatty and fibrotic degeneration of organs due to enforced lipid and specifically triglyceride accumulation and subsequent activation of profibrotic pathways may also be prevented and/or treated by applying the compounds or pharmaceutical composition of the present invention. Such conditions and diseases encompass Non-Alcoholic Steatohepatitis (NASH) and chronic cholestatic conditions in the liver, Glomerulosclerosis and Diabetic Nephropathy in the kidney, Macula Degeneration and Diabetic Retinopathy in the eye and Neurodegenerative diseases such as Alzheimer's Disease in the brain or Diabetic Neuropathies in the peripheral nervous system.

In practical use, the compounds of the present invention can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

Because of their ease of administration, tablets and capsules represent the most advantageous oral dosage unit form in which case solid pharmaceutical carriers are obviously employed. If desired, tablets may be coated by standard aqueous or non-aqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally as, for example, liquid drops or spray.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

The compounds of the present invention may also be administered parenterally. Solutions or suspensions of these active compounds can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils.

Any suitable route of administration may be employed for providing a mammal, especially a human, with an effective dose of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably compounds of the present invention are administered orally.

The effective dosage of active ingredient employed may vary depending on the particular compound employed, the mode of administration, the condition being treated and the severity of the condition being treated. Such dosage may be ascertained readily by a person skilled in the art.

When treating or preventing FXR mediated conditions for which compounds of the present invention are indicated, generally satisfactory results are obtained when the compounds of the present invention are administered at a daily dosage of from about 0.1 milligram to about 100 milligram per kilogram of animal body weight, preferably given as a single daily dose or in divided doses two to six times a day, or in sustained release form. For most large mammals, the total daily dosage is from about 1.0 milligrams to about 1000 milligrams, preferably from about 1 milligram to about 50 milligrams. In the case of a 70 kg adult human, the total daily dose will generally be from about 7 milligrams to about 350 milligrams. This dosage regimen may be adjusted to provide the optimal therapeutic response.

Some abbreviations that appear in this application are as follows.

**Abbreviations**

| **Abbreviation** | **Designation** |
|---|---|
| DCM | Dichloromethane |
| DIAD | Diisopropyl azodicarboxylate |
| DMF | Dimethylformamide |
| DMSO | Dimethylsulfoxide |
| EA | Ethyl acetate |
| EDTA | 2-[2-(Bis(carboxymethyl)amino)ethyl-(carboxymethyl)amino]acetic acid |
| ESI | Electrospray ionisation |
| FXR | Farnesoid X receptor |
| GST | Glutathione-S-transferase |
| HPLC | High-performance liquid chromatography |
| IPTG | Isopropyl β-D-1-thiogalactopyranoside |
| LBD | Ligand binding domain |
| LC/MS | Liquid chromatography - mass spectroscopy |
| NMR | Nuclear magnetic resonance |
| PE | Petroleum ether |
| po | Perorally |
| *R*f | Retention factor |
| SDS | Sodium dodecyl sulfate |
| THF | Tetrahydrofurane |
| TLC | Thin layer chromatography |
| TMS | Tetramethyl silane |

The compounds of the present invention can be prepared according to the procedures of the following Schemes and Examples, using appropriate materials and are further exemplified by the following specific examples. Moreover, by utilizing the procedures described herein, in conjunction with ordinary skills in the art, additional compounds of the present invention claimed herein can be readily prepared. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds. The instant compounds are generally isolated in the form of their pharmaceutically acceptable salts, such as those described above.

The amine-free bases corresponding to the isolated salts can be generated by neutralization with a suitable base, such as aqueous sodium hydrogen carbonate, sodium carbonate, sodium hydroxide and potassium hydroxide, and extraction of the liberated amine-free base into an organic solvent, followed by evaporation. The amine-free base, isolated in this manner, can be further converted into another pharmaceutically acceptable salt by dissolution in an organic solvent, followed by addition of the appropriate acid and subsequent evaporation, precipitation or crystallization. The carboxylic free acids corresponding to the isolated salts can be generated by neutralization with a suitable acid, such as aqueous hydrochloric acid, sodium hydrogen sulfate, sodium dihydrogen phosphate, and extraction of the liberated carboxylic-free acid into an organic solvent, followed by evaporation. The carboxylic acid, isolated in this manner, can be further converted into another pharmaceutically acceptable salt by dissolution in an organic solvent, followed by addition of the appropriate base and subsequent evaporation, precipitation or crystallization.

An illustration of the preparation of compounds of the present invention is shown below. Unless otherwise indicated in the schemes, the variables have the same meaning as described above. The examples presented below are intended to illustrate particular embodiments of the invention. Suitable starting materials, building blocks and reagents employed in the synthesis as described below are commercially available from Sigma-Aldrich Chemie GmbH, Munich, Germany, from Acros Organics, Belgium or from Fisher Scientific GmbH, 58239 Schwerte, Germany, for example, or can be routinely prepared by procedures described in the literature, for example in "March's Advanced Organic Chemistry: Reactions, Mechanisms, and Structure", 5th Edition; John Wiley & Sons or Theophil Eicher, Siegfried Hauptmann "The Chemistry of Heterocycles; Structures, Reactions, Synthesis and Application", 2nd edition, Wiley-VCH 2003; Fieser et al. "Fiesers' Reagents for organic Synthesis" John Wiley & Sons 2000.

In formulas of general synthesis schemes depicted below
- E_{L} is: a leaving group; such as halogen, OH, O-aryl, O-sulfonylalkyl or O-sulfonylaryl;
- L_{A} is: halogen, OH, NH₂, NHC(O)Oalkyl, N(R⁶)H, nitro, azido, CN, COCI, COF, CHO, CH₂OH, COOH, C(O)O-alkyl, C(O)O-aryl, C(O)O-hetaryl, OH, O-alkyl, CH₂OH or CH₂-halogen;
- L_{B} is: halogen, OH, NH₂, NHC(O)Oalkyl, N(R⁶)H, CH₂OH or CH₂-halogen;
- L_{C} is: CHO, halogen, OH, CH₂OH, CH₂NH₂, CH₂-N(R⁶)H, or CH₂-halogen.

R⁶ is as defined in the claims.

In preferred embodiments of the synthesis method
- E_{L} is: halogen or OH;
- L_{A} is: halogen, NH₂, N(R⁶)H, nitro, or OH;
- L_{B} is: halogen, NH₂ or N(R⁶)H;
- L_{C} is: CHO, CH₂NH₂, CH₂N(R⁶)H or CH₂-halogen.

In an even more preferred synthesis method
- E_{L} is: halogen or OH;
- L_{B} is: NH₂ or N(R⁶)H;
- L_{C} is: CHO or CH₂-halogen.

Isoxazole compounds of the general formula IVa are known in the art and, to the extent not commercially available, are readily synthesized by standard procedures commonly employed in the art (Scheme 1), for example by combining an acetylene compound of general formula Villa with an alpha-chlorooxime compound of formula IIa as described by Quilio et al., Gazz. Chim. Ital. 1937, 67, 589. Alternatively, if R¹ and R² are together carbonyl compounds IVa are accessible by reacting alpha-chlorooxime of formula IIa with 1,3-dicarbonyl compound Va as described, for example, by Maloney et al., J. Med. Chem. 2000, 43(16), 2971-2974 and by Doley et al, J. Chem. Soc. 1963, 5838-5845. Another method for preparing compounds of formula IVa involves combining an acetylene compound of formula Villa with nitrile oxides of general formula Via as exemplified by Himbert et al., Liebigs Ann. Chem, 1990, 4, 403-407 and in Beyer et al., Justus Liebigs Ann Chem 1970, 45-54.

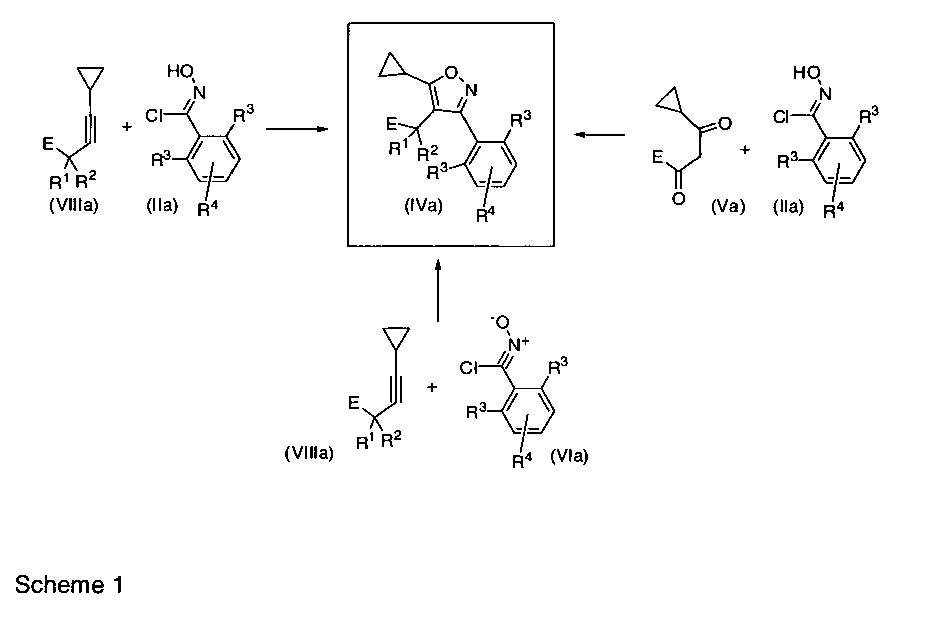

In Scheme 1 shown above, the variants R¹ and R² are defined as in the claims. Additionally, R¹ and R² may be combined to represent a carbonyl group together with the carbon atom to which they are attached. The variants R³ and R⁴ are defined as in the claims. The variant E denotes a OH group or the group E_{L} having the meaning defined above.

Compounds of formula IVb (Scheme 2) are known in the art and, to the extent not commercially available, readily synthesized by standard procedures commonly employed in the art, for example by the procedures described by J. Piet et al., Bull. Soc. Chim. Belg., 1996, 105(1), 33-44 and by A. Cwiklicki et al., Arch. Pharm. 2004, 337(3), 156-163.

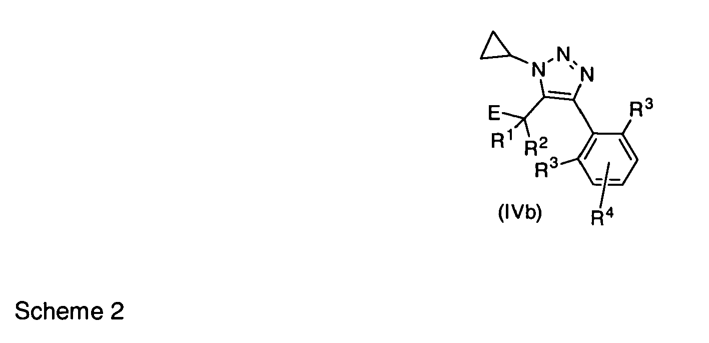

The variants of compounds of formula IVa-IVb may optionally be further transformed into other variants of compounds of formula IVa-IVb by using derivatisation reactions known to the person skilled in the art, which are described in the literature, for example in: T. Eicher, S. Hauptmann "The Chemistry of Heterocycles; Structures, Reactions, Synthesis and Application", 2nd edition, Wiley-VCH 2003 (hereafter referred to as *Eicher*); "March's Advanced Organic Chemistry: Reactions, Mechanisms, and Structure", 5th Edition; John Wiley & Sons (hereafter referred to as *March*); Larock "Comprehensive Organic Transformations", VCH Publishers, New York, N.Y. 1989 (hereafter referred to as *Larock*); Fieser et al. "Fiesers' Reagents for organic Synthesis" John Wiley & Sons 2000 (hereafter referred to as *Fieser*).

Compounds of general formula IXa and IXb as depicted in Scheme 3 are known in the art and, to the extent not commercially available, readily synthesized by standard procedures commonly employed in the art, for example by the procedures described by the literature cited above. For specific preparations see examples 1-4.

Compounds of general formula IVa, IVb, IXa, IXb, XII, XIII and XIV may optionally be equipped with a temporarily attached protecting group remaining in the compound after its conversion. In later course of the synthesis sequence the protecting group is removed as taught in: T. Greene, P. Wuts "Protective groups in organic synthesis" 3rd ed. Wiley & Sons 1999 (hereafter referred to as *Greene*).

A typical synthesis for the compounds of general formula (I) involves a multistep synthesis sequence as depicted in Scheme 3 and in Scheme 4. Starting from heterocyclic intermediates of general formula IVa or IVb, two synthetic routes are envisaged for the first step resulting in an intermediate of general formula XII.

In step A1, a suitable compound of general formula IXa, equipped with an nucleophilic OH group is dissolved or suspended in a suitable solvent, preferably but not limited to dimethylformamide, tetrahydrofurane, benzene, toluene, dichloromethane or ether and, if advantageous, a suitable base is optionally added, including but not limited to sodium methoxide, potassium methoxide, potassium carbonate, sodium hexamethyldisilazane, lithium diisopropylamide, *n-*butyllithium or an amine base such as diisopropylethylamine, followed by the addition of a compound of general formula IVa-IVb, equipped with a suitable leaving group E_{L}. If a base is required, it is typically employed in a one to one ratio. However, as the skilled artisan would appreciate, a molar excess, usually in about 1-3 fold molar excess is acceptable. The reactants are typically combined at a temperature from about 0°C to about 100°C, preferably at room temperature and the resulting mixture is typically agitated for from about 5 minutes to about 48 hours. In case where E_{L} is a poor leaving group (OH for example), it needs to be activated by adding activating reagents to the reaction mixture such as MeSO₂Cl, CF₃(SO₂)₂O or Mitsunobu reagents diisopropyldiazenedicarboxylate and triphenylphosphine, for example, as shown in example 1.

Preferably, in step A1 the leaving group E_{L} is chlorine, bromine or OH. In case where E_{L} is chlorine or bromine, the reactants of general formula IXa are dissolved or suspended in a suitable solvent, preferably tetrahydrofurane, DMF or methanol and typically 1 to 2 equivalent of a suitable base, such as sodium hydride or sodium methanolate are added. Subsequently a compound of general formula IVa-IVb is added and the resulting mixture is typically agitated for from about 5 minutes to about 48 hours. Reaction temperatures may range from -10°C to +60°C, typically from -10°C to +10°C. In case where E_{L} is OH, the reactants of general formula IVa-IVb and IXa are dissolved or suspended in a suitable solvent, preferably benzene or toluene, and 1 to 2 equivalents triphenylphosphine and diisopropyldiazenedicarboxylate (DIAD) are added. The reactants are typically combined at a temperature from about 0°C to about 50°C, preferably at room temperature. The reaction times are typically 1 h to 12h. The solvents are usually removed by distillation at temperatures typically ranging from 10°C to 50°C. The crude product is optionally purified by column chromatography and other purification methods known in the art.

In step A2, a suitable compound of general formula IXb, equipped with a suitable leaving group E_{L}, is combined with a compound of general formula IVa-IVb under similar conditions as applied in step A1.

Most preferably, in step A2 the leaving group E_{L} in compound of general formula IXb is chlorine. In this case the reactants of general formula IVa-IVb are dissolved or suspended in a suitable solvent, preferably tetrahydrofurane, DMF or methanol and typically 1 to 2 equivalents of a suitable base, such as sodium hydride or sodium methanolate are added. Subsequently a compound of general formula IXb is added and the resulting mixture is typically agitated for about 1h to 12h. Reaction temperatures may range from -10°C to +60°C, typically from -10°C to +10°C.

The starting materials and products of step A1 and step A2 may optionally be equipped with a protecting group remaining in the compound which needs to be removed in an additional step as taught in *Greene*.

In an optional step B the variants of compounds of formula XII may optionally be further transformed into other variants of compounds of formula XIV by using derivatisation reactions known to the person skilled in the art, which are described in *Greene, Eicher* and *Larock* Such derivatisation reactions are thought to turn a functional group L_{A} in formula XII into a functional group moiety L_{B} in formula XIV, which is able to undergo a reaction with moiety L_{C} in compound of formula XIII as depicted in step C (Scheme 5). General methods for functional group interconversions are described in *Larock.* In one example of step B, L_{A} is a nitro group, which is reduced into an amino group L_{B}. In another example, L_{A} is a nitro group, which is converted into a bromine L_{B} by reduction, subsequent diazotation and subsequent substitution by a bromide.

The starting materials and products of step B may optionally be equipped with a protecting group remaining in the compound which needs to be removed in an additional step as taught in *Greene*.

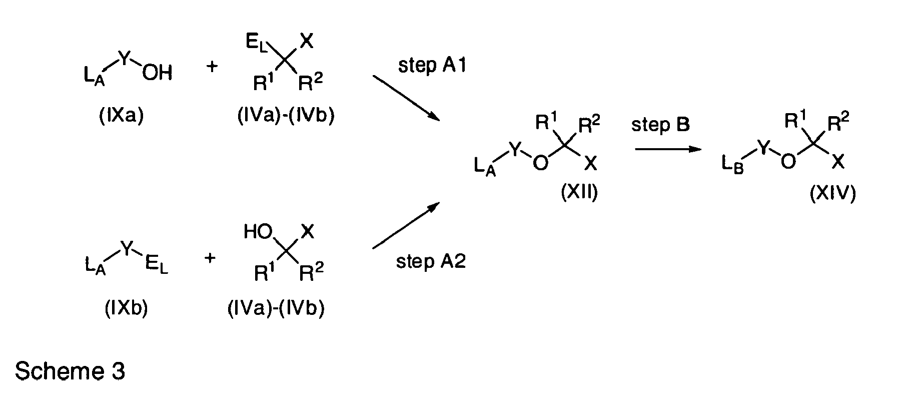

In step C of Scheme 4, a suitable compound of general formula XIII bearing a functional group L_{C} is dissolved or suspended in a suitable solvent, preferably but not limited to dimethylformamide, acetonitrile, tetrahydrofurane, benzene, toluene, dichloromethane or ether and, if advantageous, a suitable base is optionally added, including but not limited to sodium methoxide, potassium methoxide, potassium carbonate, sodium hexamethyldisilazane, lithium diisopropylamide, *n*-butyllithium or an amine base such as diisopropylethylamine, triethylamine or N-methylmorpholine. A compound of general formula XIV, equipped with a functional group L_{B} is added. It is contemplated that variants of compounds of general formula XIII and variants of compounds of general formula XIV are selected in a way that the synthetic combination of functional group L_{C} with functional group L_{B} results in a moiety L as defined in the description above. If a base is required, it is typically employed in a one to one ratio. However, as the skilled artisan would appreciate, a molar excess, usually in about 1 to 3 fold molar excess is acceptable. The reactants are typically combined at a temperature from about 0°C to about 100°C, preferably at room temperature and the resulting mixture is typically agitated for from about 5 minutes to about 48 hours. In case where L_{B} and L_{C} are groups of low reactivity that do not combine under the conditions described above, the use of an activating agent may be necessary. In one embodiment where L_{B} is NH₂ and L_{C} is CHO a condensing agent such as BF₃xEt₂O might be required for driving the initial condensation step to completion and the intermediate imine is subsequently reduced to an aminomethyl linker group L.

The products of step C may optionally be equipped with a protecting group remaining in the compound which needs to be removed in an additional step as taught in *Greene*. The variants of compounds of general formula I may optionally be further transformed into other variants of compounds of general formula I by using derivatisation reactions known to the person skilled in the art, which are described in *Greene, Eicher* and *Larock*. Specific examples of such functional group interconversions can be found in the examples section.

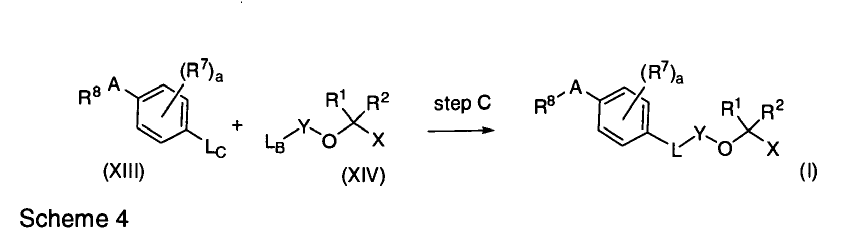

The skilled artisan will appreciate, that the synthesis steps described above may be optionally carried out in an alternative synthesis sequence, i.e. a compound of general formula XIII may be combined with a compound of general formula IX by the techniques mentioned above and the resulting intermediate is subsequently combined with a compound of general formula IVa-IVb to give products of general formula (I). The particular order of steps required to produce the compounds of formula I is dependent upon the particular compound being synthesized, the starting compound, and the relative lability of the substituted moieties.

Most preferred examples of the synthesis procedures are outlined in Scheme 5 where
- R³ is: hydrogen, fluorine, chlorine, CF₃ or OCF₃;
- R⁴ is: hydrogen;
- E_{L} is: chlorine, bromine, or OH;
- L_{A} is: nitro;
- L_{B} is: NH₂;
- L_{C} is: CHO;
- Y is: Y¹;
- L is: -CH₂-N(R⁶)-;
- R⁶ is: C₁-C₃-alkyl;
- A is: a bond;
- R⁷ is: absent, C₁-C₆ alkyl or O-C₁-C₆ alkyl;
- R⁸ is: COOR⁹.

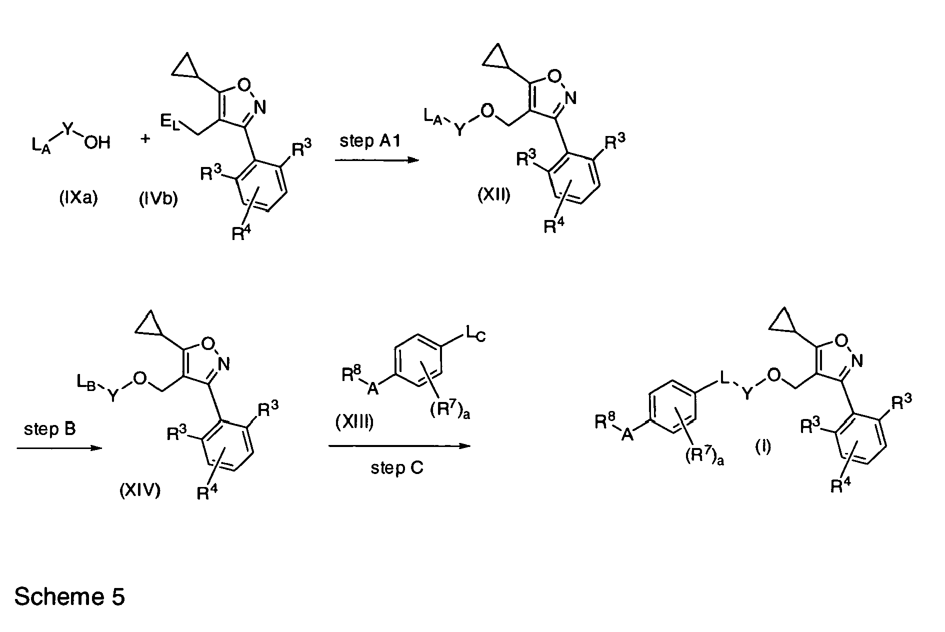

In step A1 for the synthesis of most preferred embodiments of the invention (Scheme 5), a suitable compound of general formula (IVb) is dissolved or suspended in a suitable solvent, preferably tetrahydrofurane, methanol, benzene or toluene. In case where E_{L} is chlorine or bromine, a base is added, for example sodium hydride or sodium methanolate or the like. In case where E_{L} is OH, 1 to 2 equivalents triphenylphosphine and diethyldiazenedicarboxylate (DEAD) are added. A compound of general formula IXa is added and the reactants are typically combined at a temperature ranging from about 0°C to about 50°C, preferably at room temperature. The reaction times are typically 1 to 24h. The solvents are usually removed by distillation at temperatures typically ranging from 10°C to 50°C. The crude product of general formula XII is optionally purified by extraction methods and/or column chromatography and other purification methods known in the art.

In step B of most preferred embodiments of the invention, a nitro group L_{A} in a compound of general formula XII is reduced to give an amino group L_{B} being part of a compound of general formula XIV. Several reduction methods are suitable as described in *Larock,* for instance hydrogenation in presence of a hydrogenation catalyst such as palladium on charcoal, or using sodium borohydride in methanol and nickel chloride as catalyst. A reduction using zinc and acetic acid may be carried out as follows: a suitable compound of general formula XII is dissolved or suspended together with zinc powder in a suitable solvent, preferably an alcohol such as methanol and an acid, preferably acetic acid is added. The reactants are typically combined at a temperature from about 0°C to about 50°C, preferably at room temperature, until sufficient conversion is detected by methods known in the art, such as TLC or HPLC. The reaction times typically range from 1 to 24h. Optionally, solid byproducts may then be removed by filtration. Volatiles are removed by methods known in the art, such as distillation, for example. The crude product of general formula XIV is optionally purified by extraction methods and/or column chromatography and other purification methods known in the art.

In step C of most preferred embodiments of the invention, the amino group in the compound of general formula XIV is combined with an aldehyde moiety L_{C} in compounds of general formula XIII and the intermediate imine compound is reduced by a hydride donor reagent into a compound of general formula I. Several methods are suitable for this transformation as described in *Larock,* including condensation to an intermediate imine using acetic acid as a catalyst or using BF₃xEt₂O as dehydrating agent followed by Na(CN)BH₃ or NaBH(OAc)₃ as reducing agent.

As mentioned above, compounds of general structure I may further be converted into other variants of the same general structure by single or multistep functional group interconversions such as described in *Larock.*

In most preferred embodiments, an aminomethyl group L is N-alkyated by an alkyl halogen, preferably methyl iodide. In a typical procedure, the compound of generally formula I is dissolved or suspended in a suitable solvent, for example THF at a temperature ranging from -10 to + 30°C, typically at 0°C and a base, typically NaH is added and the mixture is optionally agitated until deprotonation has progressed sufficiently. An alkyl halogen is added and agitation is continued at a temperature ranging from -10°C to +80°C, typically room temperature until conversion into the alkylation product is sufficient. The volatiles are removed by methods known in the art and the crude product is optionally further purified by the generally accepted methods, such as extraction and/or chromatography.

In other most preferred embodiments, a carboxylic ester group R⁸ in compound of formula I is saponified to give the corresponding carboxylic acid R⁸ of formula I. In a typical procedure, compound of formula I is dissolved in an suitable solvent, such as an alcohol or an ether, preferably methanol, optionally containing 0-50% of water, together with 1 to 10 equivalents, preferably 1 to 2 equivalents of a base, preferably NaOH or LiOH. The reactants are typically combined at a temperature from about 0°C to about 80°C, preferably from room temperature to 60°C, until sufficient conversion is detected by methods known in the art, such as TLC or HPLC. The reaction times typically range from 1 to 24h. The crude product of general formula I is optionally purified by extraction methods and/or column chromatography and other purification methods known in the art.

Unless otherwise noted, all non-aqueous reactions were carried out either under an argon or nitrogen atmosphere using commercial dry solvents.

LC/MS analysis was performed using a Agilent LC/MSD 1200 Series (Column: Welchrom XB-C18 (50x4.6 mm, 5 µm) operating in ESI (+) or (-) ionization mode; T = 30°C; flow rate = 1.5 ml/min.

¹H-NMR spectra were recorded on a Varian MercuryPlus 300 MHz spectrometer (method A) or on a Bruker 300MHz spectrometer (method B). Chemical shift values are given in ppm relative to TMS, with the residual solvent proton resonance as internal standard. TLCs were taken using Merck (silica gel Si-60 F254, 0.25 mm) plates using solvents as indicated.

### Preparation of intermediates

Preparation 1

### [5-Cyclopropyl-3-(2,6-dichlorophenyl)isoxazol-4-yl]methanol

The compound was synthesized by the method published in WO2007092751 (page 10, preparation 2).

### Preparation 2

### 5-Nitro-6-(trifluoromethyl)pyridin-2-ol

Step 1: The reaction was performed in three identical experiments of equal size: 2-chloro-6-methoxy-3-nitropyridine (altogether 15.0 g, 79.6 mmol), copper(I)-iodide (6.06 g, 31.8 mmol, 1.2 eq.) and potassium fluoride (3.08 g, 53.0 mmol, 2.0 eq.) were suspended in DMF (30 ml), and methyl chlorodifluoroacetate (7.1 ml, 66.3 mmol, 2.5 eq.) was added. The mixture was stirred at 125°C for 13 h and was allowed to cool to room temperature. The reaction mixture was poured into a mixture of conc. aq. NH₃-solution (140 ml) and saturated aq. NH₄Cl-solution (210 ml), and the deep-blue solution was stirred for 1.5 h at room temperature. Extraction with EA (3x150ml) was followed by washing of the combined organic layer with ¼-saturated NaCl-solution (100 ml) and brine (2×50 ml), drying over Na₂SO₄ and evaporation of the solvent. The crude product (20 g) was purified by flash chromatography (eluent hexane/DCM 6:1 to 4:1) to give 14.0 g (62.9 mmol, 79%) of 6-methoxy-3-nitro-2-(trifluoromethyl)pyridine as a yellow oil.
TLC (hexane/DCM 4:1) *R*f: 0.29. ¹H-NMR (method B, DMSO-d₆): 8.53 (dd, J = 0.5, 9.0, 1H), 7.39 (dd, J = 0.4, 9.0, 1H), 4.02 (s, 3H).
Step 2: 6-methoxy-3-nitro-2-(trifluoromethyl)pyridine from step 1 (14.0 g, 62.9 mmol) was dissolved in a solution of 33% HBr in acetic acid (120 ml) at room temperature. The flask was equipped with a bubble counter, and the solution was stirred at 40°C for 14 h. After cooling to room temperature, the volatiles were removed under vacuum and the residue was coevaporated with EA (2x100ml). The residual yellow oil was adsorbed on silica gel and purified by repeated flash chromatography (eluent: hexane/EA 9:1 to 2:1, then hexane/EA 4:1 to 1:2) to give 5-nitro-6-(trifluoromethyl)pyridin-2-ol as a pale yellow solid (9.52 g, 45.7 mmol, yield: 73%).
TLC (hexane/EA 4:1) Rf: ∼0.10. ¹H-NMR (method B, DMSO-d₆): 13.1 (br.s,1H), 8.43 (d, J = 9.0, 1H), 7.11 (d, J = 9.0, 1H).

### Preparation 3

### Methyl 4-formyl-2,6-dimethylbenzoate

Step 1: A solution of 2,5-dibromoxylene (2.0 g, 7.6 mmol) in DCM (20 ml) was treated with *n*-BuLi (2.5 M solution in hexane, 3 ml, 1.0 equiv.) at -78°C. After stirring for 1h, DMF (1.8 ml, 23 mmol, 3 eq.) was added and the solution was allowed to warm to room temperature. It was acidified to pH 2 with 5% hydrochloric acid and extracted with diethyl ether (3x50 ml). The combined organic phase was dried and evaporated and the residue was purified by column chromatography using PE:EA = 10:1 as eluent to give 4-bromo-3,5-dimethylbenzaldehyde (0.76 g, yield: 47%).

Step 2: A solution of 4-bromo-3,5-dimethylbenzaldehyde (0.76 g, 3.6 mmol) in dry DMF (5 ml) was added dry methanol (20 ml) followed by NaOAc (0.6 g, 7.2 mmol, 2 eq.) and Pd(dppf)₂Cl₂ (0.3 g, 0.36 mmol, 0.1 eq.). The reaction mixture was heated to 90°C under a carbon monoxide atmosphere for 20h. The reaction mixture was cooled to ambient temperature and evaporated. The residue was purified by column chromatography using PE:EA = 10:1 as eluent to give methyl 4-formyl-2,6-dimethylbenzoate (0.3 g, yield: 43%).
¹H NMR (method A, CDCl₃, 300MHz): δ= 2.37 (s, 6H), 2.95 (s, 3H), 7.54-7.55 (m, 2H), 9.96 (s.1H).

### Preparation 4

### [3-(2,6-Dichloro-phenyl)-5-isopropyl-isoxazol-4-yl]-methanol

The compound was synthesized by the method published in: P. Maloney et al. "Identification of a chemical tool for the orphan nuclear receptor FXR" J. Med. Chem. 2000, 43(16), 2971-2974.

### Preparation of Comparative Compounds

Preparation 5 (Compound A)

### 3-(((6-((5-Cyclopropyl-3-(2,6-dichlorophenyl)isoxazol-4-yl)methoxy)-2-(trifluoromethyl)pyridin-3-yl)(methyl)amino)methyl)benzoic acid

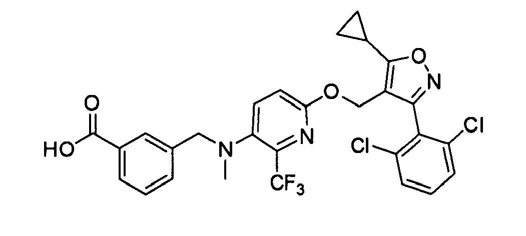

Step 1: A solution of 6-((5-cyclopropyl-3-(2,6-dichlorophenyl) isoxazol-4-yl)methoxy)-2-(trifluoromethyl)pyridin-3-amine (synthesised as described for example 1, step 2, 5.08 g, 11.4 mmol) and methyl 3-formylbenzoate (2.05 g, 12.5 mmol) in THF (150 ml) was cooled to 5 °C and BF₃xEt₂O (8.07 g, 5 eq) was added dropwise. The reaction was stirred at 25°C for 12 h, cooled to 5°C, and NaCNBH₃ (0.72 g) was added in portions. After 2h, the reaction was quenched with NaHCO₃ (100ml), while the temperature was kept below 10°C. The THF layer was separated, and the aqueous layer was extracted with EA (2x100 ml). The combined organic layer was washed with brine (200 ml), dried over Na₂SO₄, filtered and concentrated. The crude material was purified by flash chromatography using PE/EA mixtures as eluent to yield methyl 3-((6-((5-cyclopropyl-3-(2,6-dichlorophenyl) isoxazol-4-yl)methoxy)-2-(trifluoromethyl) pyridin-3-ylamino)methyl)benzoate (6.50 g, 11.0 mmol, yield: 89%).

Step 2: The product from step 1 was N-methylated by an analog procedure as described for example 1, step 4, to give methyl 3-(((6-((5-cyclopropyl-3-(2,6-dichlorophenyl)isoxazol-4-yl)methoxy)-2-(trifluoromethyl)pyridin-3-yl)(methyl)amino)methyl) benzoate in 75% yield.

Step 3: The product from step 2 was saponified by the analog procedure as described for example 1, step 5, to give 3-(((6-((5-cyclopropyl-3-(2,6-dichlorophenyl) isoxazol-4-yl)methoxy)-2-(trifluoromethyl)pyridin-3-yl)(methyl)amino)methyl)benzoic acid in 90% yield.

¹H NMR (method A, 300 MHz, CDCl₃) δ: 1.10-1.30 (m, 4H), 2.36-2.42 (m, 1H), 2.54 (s, 3H), 4.01 (s, 2H), 5.23 (s, 2H), 6.76-6.80 (m, 1H), 7.27-7.40 (m, 3H), 7.41-7.48 (m, 1H), 7.62-7.73 (m, 1H), 8.01-8.10 (m, 1H).
LC-MS: rt 4.36 min; m/z [M+H]⁺ 590.1 (calculated: 590.1).

### Preparation 6 (Compound B, prior art)

### 4-(((6-((3-(2,6-Dichlorophenyl)-5-isopropylisoxazol-4-yl)methoxy)-2-(trifluoromethyl)pyridin-3-yl)(methyl)amino)methyl)benzoic acid

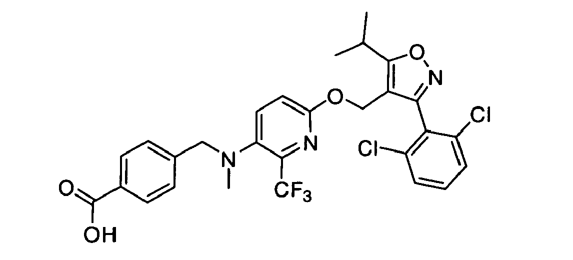

Step 1: 5-Nitro-6-(trifluoromethyl)pyridin-2-ol from preparation 2 (0.396 g, 1.9 mmol), [3-(2,6-dichloro-phenyl)-5-isopropyl-isoxazol-4-yl]-methanol from preparation 4 (0.653 g, 2.3 mmol), triphenylphosphine (0.898 g, 3.4 mmol) and benzene (15 ml) were placed in a reaction vessel followed by dropwise addition of diisopropyldiazenedicarboxylate (0.74 g, 3.7 mmol). The reaction mixture was stirred at room temperature for 3 h and the solvent was removed under reduced pressure affording a crude product which was further purified by column chromatography on silica (PE : EA 10:1) to give 6-[3-(2,6-dichloro-phenyl)-5-isopropyl-isoxazol-4-ylmethoxy]-2-trifluoromethyl-3-nitro-pyridine (0.66 g, yield 73%) as pale yellow powder.

Step 2: Zinc powder (0.21 g, 3.1 mmol) was added to a vigorously stirred suspension of 6-[3-(2,6-dichloro-phenyl)-5-isopropyl-isoxazol-4-ylmethoxy]-2-trifluoromethyl-3-nitro-pyridine (0.150 g, 0.310 mmol) in methanol (4ml), followed by the dropwise addition of acetic acid (0.084 g, 1.4 mmol). The resulting mixture was stirred for 2 h at 50°C and filtered through a 2 cm layer of silica (eluent methanol). The eluent was concentrated under reduced pressure and the residue dissolved in ethyl acetate (20 ml). This solution was filtered by paper filter, washed with 10% aqueous K₂CO₃ (5 ml), water (5 ml), dried over anhydrous Na₂SO₄ and evaporated to give 6-[3-(2,6-dichlorophenyl)-5-isopropyl-isoxazol-4-ylmethoxy]-2-trifluoromethyl-pyridin-3-ylamine (0.124 g, yield 90%) as a yellowish oil.

Step 3: The product derived from the previous step (0.579 g, 1.3 mmol) and 4-formyl-benzoic acid methyl ester (0.639 g, 3.9 mmol) was dissolved in 1,2-dichloroethane (20 ml) and glacial acetic acid (0.47 g, 7.8 mmol) was added. The reaction mixture was stirred at room temperature for 1 h, sodium triacetoxy-borohydride (1.24 g, 5.9 mmol) was added and stirring was continued for 24 h. A saturated aqueous solution of sodium hydrogencarbonate (15 ml) was added and the aqueous layer was extracted with ethyl acetate (3×20 ml). The combined organic extracts were washed with brine, dried over sodium sulfate and concentrated at reduced pressure. The residue was purified by HPLC (reversed phase, eluent: acetonitrile-water) to provide 4-({6-[3-(2,6-dichloro-phenyl)-5-isopropyl-isoxazol-4-ylmethoxy]-2-trifluoromethyl-pyridin-3-ylamino}-methyl)-benzoic acid methyl ester as a white powder (0.38 g, 49%).

Step 4: The product derived from previous step 3 (0.076 g, 0.128 mmol) was dissolved in 1,2-dichloroethane (5 ml) and paraformaldehyde (0.023 g, 0.77 mmol), acetic acid (0.046 g, 0.77 mmol) and sodium triacetoxyborohydride (0.163 g, 0.77 mmol) was added and the reaction mixture was stirred at room temperature for 24 h. The mixture was diluted with saturated aqueous sodium hydrogencarbonate and extracted with three portions of ethyl acetate. The combined extracts were dried over sodium sulfate and concentrated at reduced pressure. The residue was purified by preparative TLC on silica (eluent hexane-ethyl acetate 5:1) to obtain 4-[({6-[3-(2,6-dichloro-phenyl)-5-isopropyl-isoxazol-4-ylmethoxy]-2-trifluoromethyl-pyridin-3-yl}-methyl-amino)-methyl]-benzoic acid methyl ester (0.046 g, yield 59%) as a colourless gum.

Step 5: A suspension of the product derived from previous step 4 (0.046 g, 0.076 mmol) in methanol (5 ml) was treated with sodium hydroxide (0.03 g, 0.76 mmol) and water (0.3 ml) and the reaction mixture was stirred at room temperature for 7 h. The solvent was removed *in vacuo* and the residue was diluted with water (1 ml). The resulting solution was acidified with acetic acid to pH 6 and the formed precipitate was filtered, washed with water (3x5 ml) and dried on air to obtain the title product as a white powder (0.030 g, yield 66%).
M.p. 96.1-97.1 °C.
¹H-NMR (400 MHz, DMSO-d₆); δ (ppm) 1.31 (6H, d), 2.48 (3H, s), 3.41-3.57 (1H, m), 4.00 (2H, s), 5.19 (2H, s), 6.81 (1H, d), 7.33 (2H, d), 7.40-7.52 (3H, m), 7.84 (2H, d), 7.96 (1H, d).
LC-MS: rt 2.21 min; m/z [M-H]⁻ 592.2 (calculated: 592.1).

### Preparation 7 (Compound C, prior art)

### 3-(((6-((3-(2,6-Dichlorophenyl)-5-isopropylisoxazol-4-yl)methoxy)-2-(trifluoromethyl)pyridin-3-yl)(methyl)amino)methyl)benzoic acid

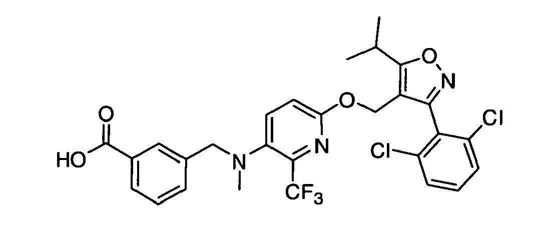

Step 1: A solution of the product derived from preparation 5 step 2 (6-[3-(2,6-dichlorophenyl)-5-isopropyl-isoxazol-4-ylmethoxy]-2-trifluoromethyl-pyridin-3-ylamine, 5.08 g) and methyl 3-formylbenzoate (2.05 g, 1.1 eq) in THF (150 ml) was cooled to 5 °C and BF₃xEt₂O (8.07 g, 5 eq) was added dropwise. The reaction was stirred at 20 °C for 12h, cooled to 5 °C and NaCNBH₃ (0.72 g, 1 eq) was added in portions. After stirring for 1 h at 5°C, the reaction was quenched by adding aqueous NaHCO₃ (2N, 100 ml) while the temperature was kept below 10°C. The THF layer was separated, and the aqueous phase was extracted with EA (2x100 ml). The combined organic layer was washed with 200 ml of saturated brine, dried over Na₂SO₄, filtered and concentrated. The crude product was purified by silica gel chromatography using a gradient of PE: EA from 20:1 to 5:1 as eluent to yield methyl 3-((6-((3-(2,6-dichlorophenyl)-5-isopropylisoxazol-4-yl)methoxy)-2-(trifluoromethyl)pyridin-3-ylam ino)methyl)benzoate (6.5 g, 10.9 mmol, yield: 95%).

Step 2: The product derived from previous step 1 (0.076 g, 0.128 mmol) was dissolved in 1,2-dichloroethane (5 ml) and paraformaldehyde (0.023 g, 0.77 mmol), acetic acid (0.046 g, 0.77 mmol) and sodium triacetoxyborohydride (0.163 g, 0.77 mmol) was added. The reaction mixture was stirred at room temperature for 24 h. The mixture was diluted with saturated aqueous sodium hydrogencarbonate and extracted with three portions of ethyl acetate. The combined extracts were dried over sodium sulfate and concentrated at reduced pressure. The residue was purified by preparative TLC on silica (eluent hexane-ethyl acetate 5:1) to obtain methyl 3-(((6-((3-(2,6-dichlorophenyl)-5-isopropylisoxazol-4-yl)methoxy)-2-(trifluoromethyl)pyridin-3-yl) (methyl)amino)methyl)benzoate (0.055 g, 0.090 mmol, 70% yield) as a colourless gum.

Step 3: A suspension of the product derived from previous step 2 (0.055 g, 0.090 mmol) in methanol (5 ml) was treated with sodium hydroxide (0.03 g, 0.76 mmol) and water (0.3 ml) and the reaction mixture was stirred at room temperature for 7 h. The solvent was removed *in vacuo* and the residue was diluted with water (1 ml). The resulting solution was acidified with acetic acid to pH 6 and the formed precipitate was filtered, washed with water (3 x 5 ml) and dried on air to obtain the title product as a white powder (0.030 g, 0.051 mmol, 75%).
¹H-NMR (400 MHz, DMSO-d₆); δ (ppm) 1.40 (d, 6H), 2.52 (s, 3H), 3.40-3.56 (m, 1H), 4.00 (s, 2H), 5.19 (s, 2H), 6.76 (dd, 1H), 7.24-7.38 (m, 3H), 7.42-7.50 (m, 1H), 7.62-7.72 (m, 2H), 8.01-8.10 (m, 1H).
LC-MS: rt 4.51 min; m/z [M-H]⁻ 592.0 (calculated: 592.1).

### Example 1

### 4-{[(6-((5-Cyclopropyl-3-(2,6-dichlorophenyl)isoxazol-4-yl)methoxy)-2-(trifluoromethyl)pyridin-3-yl)(methyl)amino]methyl}benzoic acid

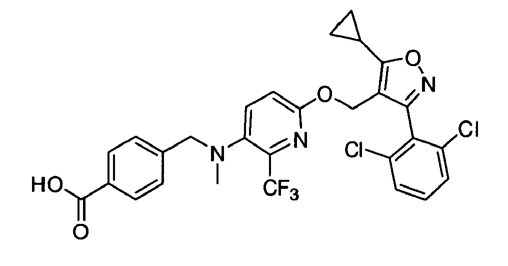

Step 1: A solution of [5-cyclopropyl-3-(2,6-dichlorophenyl)isoxazol-4-yl]methanol (see preparation 1, 480 mg, 2.29 mmol), 5-nitro-6-(trifluoromethyl)pyridin-2-ol (see preparation 2, 650 mg, 2.29 mmol) and PPh₃ (1.2 g, 4.58 mmol) in toluene (30 ml) was cooled to 0°C, DIAD (930 mg, 4.58 mmol) was added and the mixture was stirred at room temperature for 12 h. The solution was concentrated under reduced pressure and the residue was purified by silica gel column chromatography to afford 5-cyclopropyl-3-(2,6-dichlorophenyl)-4-((5-nitro-6-(trifluoromethyl)pyridin-2-yloxy)methyl) isoxazole (590 mg, 1.24 mmol, yield: 54%).

Step 2: AcOH (340 mg, 5.6 mmol) was added to a mixture of the product from step 1 (590 mg, 1.24 mmol) and Zn powder (810 mg, 12.4 mmol) in MeOH (30 ml). The mixture was stirred at 50°C for 2 h under N₂. The mixture was filtered, the filter cake was washed with MeOH and the filtrate was concentrated. A saturated solution of NaHCO₃ (20ml) was added and the mixture was extracted with EA (3x10 ml). The combined organic layer was dried over Na₂SO₄, filtered and concentrated to give 6-((5-cyclopropyl-3-(2,6-dichlorophenyl)isoxazol-4-yl)methoxy)-2-(trifluoromethyl) pyridin-3-amine (400 mg, 0.900 mmol, 73% yield).

Step 3: A solution of the product from step 2 (200 mg, 0.450 mmol, 1 eq) and methyl 4-formylbenzoate (80 mg, 0.49 mmol, 1.1 eq) in THF (6 ml) was cooled to 0 °C. BF₃-Et₂O (320 mg, 2.25 mmol, 5 eq.) was added to the reaction mixture at 0°C, and the reaction was stirred for 12 h at ambient temperature. NaCNBH₃ (28 mg, 0.450 mmol, 1 eq) was added and the reaction was stirred for 4 h at 25 °C. The reaction mixture was poured into a solution of K₂CO₃ (0.92 g) in water (3 ml) and stirred for 1 to 2 h. The organic layer was extracted with ethyl acetate (2x 25 ml) and the combined organic layer was dried over anhydrous Na₂SO₄, filtered and evaporated.

The residue was redissolved in a mixture of PE:EA 10:1 (5 ml) and passed through a silica gel plug and eluated with a mixture of PE:EA 10:1 to get of methyl 4-((6-((5-cyclopropyl-3-(2,6-dichlorophenyl)isoxazol-4-yl)methoxy)-2-(trifluoromethyl)pyridin-3-ylamino)methyl)benzoate (186 mg, 0.315 mmol, yield:70%).

Step 4: In a dry 50 ml flask, sodium hydride (60% in mineral oil, 38 mg, 0.942 mmol, 3 eq) was suspended in DMF (14 ml) at 0°C, and methyl iodide (178 mg, 1.26 mmol, 4 eq) was added. The amine product from step 3 (186 mg, 0.315 mmol) was dissolved in DMF (5 ml) and was then added slowly to the mixture at 0°C. The suspension was stirred for 4 hours. The reaction was quenched by sequencial addition of a saturated solution of NaHCO₃ (20 ml), water (25 ml) and EA (40 ml). The aqueous layer was extracted with EA (50 ml). The combined organic layer was washed consecutively with a solution of LiCl (3x 40 ml), dried over Na₂SO₄, filtered and concentrated. The crude product was purified by flash chromatography using PE:EA 5:1 as eluent to give of methyl 4-(((6-((5-cyclopropyl-3-(2,6-dichlorophenyl)isoxazol-4-yl)methoxy)-2-(trifluoromethyl)pyridin-3-yl)(methyl)amino) methyl)benzoate as a yellow gum (150 mg, 0.248 mmol, yield: 79%).

Step 5: The methyl ester from step 4 (150 mg, 0.248 mmol) was dissolved in THF (15 ml) and MeOH (5 ml) was added. LiOHxH₂O (100 mg, 2.48 mmol, 10 eq) in H₂O (10 ml) was added and the mixture was stirred at 25°C for 12h. After the reaction was completed as tested by TLC, the solvent was removed under vacuum. Water (10 ml) was added, and a 1 N HCl solution was added to adjust the acidity to pH 3. The aqueous phase was extracted with EA (3x10 ml) and the combined organic layer was dried over Na₂SO₄, filtered and concentrated to yield the title compound as a colourless solid (120 mg, 0.203, yield: 82%)
¹H NMR (method A, 300 MHz, CDCl₃) δ: 0.86 (m, 2H), 1.11-1.18 (m, 2H), 2.37-2.42 (m, 1H), 2.53 (s, 3H), 4.02 (s, 2H), 5.25 (s, 2H), 6.78 (d, 1H), 7.27-7.40 (m, 3H), 7.50 (d, 2H), 7.65 (d, 1H), 8.07 (d, 2H).
LC-MS: rt 3.49 min; m/z [M+H]⁺ 590.1 (calculated: 590.1).

### Example 2

### 4-{[(6-((5-Cyclopropyl-3-(2,6-dichlorophenyl)isoxazol-4-yl)methoxy)-2-(trifluoromethyl)pyridin-3-yl)(methyl)amino]methyl}-2-methylbenzoic acid

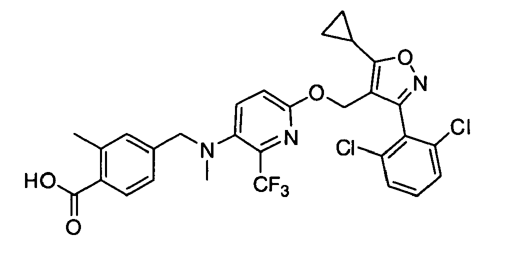

Step 1: 6-((5-Cyclopropyl-3-(2,6-dichlorophenyl)isoxazol-4-yl)methoxy)-2-(trifluoromethyl)-pyridin-3-amine (synthesised as in example 1 step 2, 197 mg, 0.444 mmol, 1 eq) was added to a solution of methyl 4-formyl-2-methylbenzoate (118 mg, 0.666 mmol, 1.5 eq) in THF (6 ml). BF₃ Et₂O (315 mg, 2.22 mmol, 5 eq) was added at 0^{°}C and the mixture was stirred for 12h at ambient temperature. The reaction mixture was poured on a 20% aqueous K₂CO₃ solution (3 ml) and stirred for 5 h and extracted with EA (4x 50 ml). The combined organic layer was dried over anhydrous Na₂SO₄ passed through a silica gel plug using a PE:EA=10:1 mixture as eluent and evaporated to yield methyl 4-((6-((5-cyclopropyl-3-(2,6-dichlorophenyl)isoxazol-4-yl)methoxy)-2-(trifluoromethyl)pyridin-3-ylamino)methyl)-2-methylbenzoate (140 mg, 0.231 mmol, yield: 52%).

Step 2: In a dry 50 ml-flask, sodium hydride (60% in mineral oil, 28 mg, 0.693 mmol, 3 eq) was suspended in DMF (4 ml) at 0°C, and methyl iodide (132 mg, 0.924 mmol, 4 eq) was added. The product from step 1 (140 mg, 0.231 mmol) was dissolved in DMF (5 ml) and the solution was added dropwise to the above mixture within 10 min at 0 °C and the suspension was stirred for 2 h at 0 °C. The reaction was quenched by sequencial addition of a saturated NaHCO₃ solution (20 ml), water (25 ml) and EA (40 ml), and the aqueous layer was extracted with EA (4x 50 ml). The combined organic layer was washed consecutively with half-saturated NaCl solution (2x 40 ml) and brine (30 ml), dried with Na₂SO₄, and concentrated. The crude product was purified by flash chromatography using PE:EA=5:1 as eluent, giving methyl 4-(((6-((5-cyclopropyl-3-(2,6-dichlorophenyl)isoxazol-4-yl)methoxy)-2-(trifluoromethyl)pyridin-3-yl)(methyl)amino) methyl)-2-methylbenzoate as a yellow oil (130 mg, 0.210 mmol, yield: 91 %).

Step 3: The product from step 2 (130 mg, 0.210 mmol) was dissolved in THF (15 ml) and MeOH (5 ml) was added folllowed by a solution of LiOHxH₂O (176 mg, 0.418 mmol, 20 eq) in H₂O (10 ml). The mixture was stirred at 30°C for 48 h and the pH was adjusted to a value of 5-6 by addition of 1 N HCl. The volatiles were evaporated and the aqueous phase was extracted with EA (3 x 20 ml). The solvent was removed under reduced pressure to yield the title compound (54 mg, 0.089 mmol, yield: 43%).
¹H NMR (method B, 300MHz, CDCl₃) δ: 1.13-1.18 (m, 2H), 1.23-1.29 (m, 2H), 2.36-2.39 (m, 1H), 2.53 (s, 3H), 2.64-2.65 (d, 3H), 3.96 (s, 2H), 5.25 (s, 2H), 6.77-6.79 (d, 1H), 7.26-7.39 (m, 5H), 7.63-7.66 (d, 1H), 8.00-8.03 (d, 1H).
LC-MS: rt 4.89 min; m/z [M+H]⁺ 606.2 (calculated: 606.1).

### Example 3

### 4-{[(6-((5-Cyclopropyl-3-(2,6-dichlorophenyl)isoxazol-4-yl)methoxy)-2-(trifluoromethyl)pyridin-3-yl)(methyl)amino]methyl}-2,6-dimethylbenzoic acid

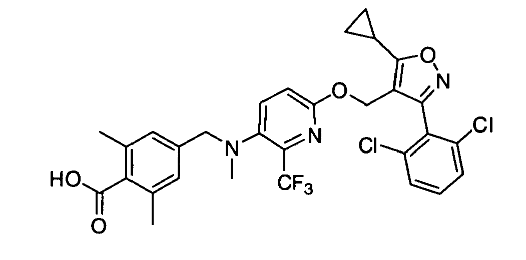

Step 1: A solution of 6-((5-cyclopropyl-3-(2,6-dichlorophenyl)isoxazol-4-yl)methoxy)-2-(trifluoromethyl)pyridin-3-amine (synthesized as described for example 1, step 2, 420 mg, 0.93 mmol) and methyl 4-formyl-2,6-dimethylbenzoate (synthesized as described in preparation 3, 200 mg, 1.02 mmol, 1.1 eq) in THF (6 ml) was cooled to 0°C. BF₃-Et₂O (660 mg, 4.65 mmol, 5 eq) was added and the reaction was stirred for 12h at ambient temperature. NaCNBH₃ (60 mg, 0.93 mmol, 1 eq) was added and stirring was continued for 4h. The reaction mixture was poured into a solution of K₂CO₃ (2 g) in H₂O (6 ml) and the mixture was stirred for 2h. The organic layer was extracted with EA (2x 25 ml) and the combined organic layer was dried over anhydrous Na₂SO₄, filtered, evaporated and passed through a silica gel plug using a PE:EA 10:1 mixture for elution to give methyl 4-((6-((5-cyclopropyl-3-(2,6-dichlorophenyl)isoxazol-4-yl)methoxy)-2-(trifluoromethyl)pyridin-3-ylamino)methyl)-2,6-dimethylbenzoate (330 mg, 0.53 mmol, 57% yield).

Step 2: In a dry 50 ml flask, sodium hydride (60% in mineral oil, 64 mg, 1.60 mmol, 3 eq) was suspended in DMF (20 ml) at 0°C, and methyl iodide (300 mg, 2.13 mmol, 4 eq) was added. The product from step 1 (330 mg, 0.53 mmol) was dissolved in DMF (5 ml) and the solution was added slowly to the NaH suspension at 0 °C and the mixture was stirred for 4 hours. The reaction was quenched by addition of a solution of NaHCO₃ (20 ml), water (25 ml) and EA (40 ml) and the aqueous layer was extracted with EA (50 ml). The combined organic layer was washed with a solution of LiCl (3 x 40 ml), dried over Na₂SO₄, filtered and concentrated. The crude product was purified by flash chromatography (eluent PE:EA = 5:1) to give methyl 4-(((6-((5-cyclopropyl-3-(2,6-dichlorophenyl)isoxazol-4-yl)methoxy)-2-(trifluoromethyl)pyridin-3-yl)(methyl)amino)methyl)-2,6-dimethylbenzoate (320 mg, 0.516 mmol, yield: 97%) as an yellow oil.

Step 3: The product from step 2 (150 mg, 0.237 mmol, 1 eq) was dissolved in a mixture of THF (5 ml) and MeOH (5 ml), a NaOH solution (2N, 40 ml) was added and the mixture was refluxed for 12h. The volatiles were removed under reduced pressure, water (10 ml) was added and the pH was adjusted to 2 to 3 by addition of 1 N HCl. The aqueous phase was extracted with DCM (3x10 ml) and the combined organic layer was dried over Na₂SO₄ and concentrated to yield the title compound (50 mg, 0.083 mmol, 35%).
¹H NMR (method B, 300 MHz, CDCl₃) δ: 1.13-1.16 (m, 2H), 1.23-1.27 (m, 2H), 2.39 (s, 6H), 2.51 (s, 3H), 3.88 (s, 2H), 5.25 (s, 2H), 6.77 (d, 1H), 7.06 (s, 2H), 7.28-7.31 (m, 1H), 7.35-7.38 (m, 2H), 7.62 (d, 1H).
LC-MS: rt 3.71 min; m/z [M+H]⁺ 620.1 (calculated: 520.1).

### Example 4

### 4-{[(6-((5-Cyclopropyl-3-(2,6-dichlorophenyl)isoxazol-4-yl)methoxy)-2-methylpyridin-3-yl)(methyl)amino]methyl}benzoic acid

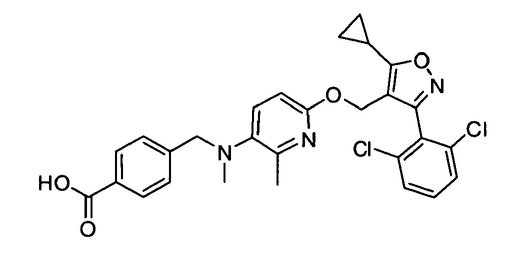

Step 1: A solution of commercially available 6-methoxy-2-methyl-3-nitropyridine (183 mg, 1.19 mmol), [5-cyclopropyl-3-(2,6-dichlorophenyl)isoxazol-4-yl]methanol (see preparation 1, 320 mg ,1.13 mmol) and PPh₃ (563 mg, 2.15 mmol) in toluene (30 ml) was cooled to 0°C, DIAD (434 mg, 2.15 mmol) was added and the mixture was stirred at room temperature for 12 h. The solution was concentrated under reduced pressure to give a crude residue which was purified by flash chromagraphy to give 5-cyclopropyl-3-(2,6-dichlorophenyl)-4-((6-methyl-5-nitropyridin-2-yloxy)methyl) isoxazole (340 mg, 0.810 mmol; 68% yield).

Step 2: AcOH (231 mg, 3.85 mmol) was added to a mixture of the product from step 1 (358 mg, 0.852 mmol) and Zn powder (557 mg, 8.56 mmol) in MeOH (30 ml). The mixture was stirred at 50°C for 2h, the solid was filtered and the residue was washed with MeOH (2 x 10 ml) and the filtrate was concentrated under reduced pressure. A saturated aqueous solution of NaHCO₃ (50 ml) was added and the mixture was extrated with EA (3x 50 ml), the combined organic phase was dried over Na₂SO₄ and concentrated to give 6-((5-cyclopropyl-3-(2,6-dichlorophenyl)isoxazol-4-yl)methoxy)-2-methylpyridin-3-amine (329 mg, 0.844 mmol, 99% yield).

Step 3: The product from step 2 (329 mg, 0.844 mmol) was added to a solution of commercially available methyl 4-formylbenzoate (209 mg, 1.27 mmol) in THF (30 ml). The reaction mixture was cooled to 0°C and BF₃ Et₂O (602 mg, 4.24 mmol) was added and the mixture was stirred for 12 h at ambient temperature. NaCNBH₃ (107 mg,1.70 mmol) was added and stirring was continued for 2 h. The reaction mixture was poured on a saturated K₂CO₃ solution (6 ml) and stirred for 5 h. The aqueous phase was extracted with EA (4x 50 ml) and the combined organic layers were dried over anhydrous Na₂SO₄, passed through a silica gel plug using PE:EA =5:1 as eluent and evaporated to give methyl 4-((6-((5-cyclopropyl-3-(2,6-dichlorophenyl)isoxazol-4-yl)methoxy)-2-methylpyridin-3-ylamino)methyl)benzoate (268 mg, 0.499 mmol, yield: 59%).

Step 4: In a dry 50 ml-flask, sodium hydride (60% in mineral oil, 59.3 mg, 1.48 mmol) was suspended in DMF (15 ml) at 0 °C and methyl iodide (281 mg ,1.96 mmol) was added. A solution of the product from step 3 (265 mg, 0.493 mmol) in DMF (15 ml) was added dropwise to the above mixture within 10 min at 0°C and the mixture was stirrred for 12 h at 20°C. A saturated NaHCO₃ solution (20 ml) was added followed by water (25 ml) and EA (40 ml) and the aqueous layer was extracted with EA (4 x 50 ml). The combined organic layer was washed consecutively with half-saturated NaCl solution (2x 40 ml) and brine (30 ml), dried over Na₂SO₄ and concentrated. The crude product was purified by flash chromatography to give methyl 4-(((6-((5-cyclopropyl-3-(2,6-dichlorophenyl)-isoxazol-4-yl)methoxy)-2-methylpyridin-3-yl)(methyl)amino) methyl)benzoate (195 mg, yield: 59%).

Step 5: The product from step 4 (190 mg, 0.345 mmol) was dissolved in a mixture of THF (15 ml) and MeOH (5 ml) and a solution of LiOHxH₂O (145 mg, 3.45 mmol) in H₂O (5 ml) was added. The mixture was stirred at 20°C for 12h and the pH was adjusted to 3 by addition of 1 N HCl. The volatiles were removed under reduced pressure and the aqueous phase was extracted by EA (3x 20 ml). The combined organic phase was dried over Na₂SO₄ and evaporated to give the title compound (183 mg, 0.340 mmol, yield: 99%).
¹H NMR (method B, 300 MHz, CDCl₃) δ: 1.04-1.15 (m, 2H), 1.22-1.30 (m, 2H), 2.32-2.42 (m, 1H), 2.44 (s, 3H), 2.53 (s, 3H), 4.00 (s, 2H), 5.15 (s, 2H), 6.39-6.41 (d, 1H), 7.25-7.30 (m, 2H), 7.35-7.40 (m, 2H), 7.42-7.45 (m, 2H), 8.03-8.08 (m, 2H).
LC-MS: rt 3.18 min; m/z [M+H]⁺ 538.1 (calculated: 538.1).

### Assays

### FRET activity assay

Determination of a ligand mediated cofactor peptide interaction to quantify ligand binding to the nuclear receptor FXR was performed as follows: Preparation of human FXR alpha ligand binding domain: The human FXRalpha LBD was expressed in E. coli strain BL21(DE3) as an N-terminally GST tagged fusion protein. The DNA encoding the FXR ligand binding domain was cloned into vector pDEST15 (Invitrogen). Expression was under control of an IPTG inducible T7 promoter. The amino acid boundaries of the ligand binding domain were amino acids 187-472 of Database entry NM_005123 (RefSeq). Expression and purification of the FXR-LBD:

An overnight preculture of a transformed E.coli strain was diluted 1:20 in LB-Ampicillin medium and grown at 30°C to an optical density of OD600=0.4-0.6. Gene expression was then induced by addition of 0.5 mM IPTG. Cells were incubated an additional 6h at 30°C, 180 rpm. Cells were collected by centrifugation (7000 x g, 7 min, room temperature). Per liter of original cell culture, cells were resuspended in 10 ml lysis buffer (50 mM Glucose, 50 mM Tris pH 7.9, 1 mM EDTA and 4 mg/ml lysozyme) and left on ice for 30 min. Cells were then subjected to sonication and cell debris removed via centrifugation (22000 x g, 30 min, 4°C). Per 10 ml of supernatant 0.5 ml prewashed Glutathione 4B sepharose slurry (Qiagen) was added and the suspension kept slowly rotating for 1 h at 4°C. Glutathione 4B sepharose beads were pelleted by centrifugation (2000 g, 15 sec, 4°C) and washed twice in wash buffer (25 mM Tris, 50mM KCl, 4 mM MgCl₂ and 1M NaCl). The pellet was resuspended in 3 ml elution buffer per liter of original culture (elution buffer: 20 mM Tris, 60 mM KCI, 5 mM MgCl₂ and 80 mM glutathione added immediately prior to use as powder). The suspension was left rotating for 15 min at 4°C, the beads pelleted and eluted again with half the volume of elution buffer than the first time. The eluates were pooled and dialysed overnight in 20 mM Hepes buffer (pH 7.5) containing 60 mM KCI, 5 mM MgCl₂ as well as 1 mM dithiothreitol and 10% (v/v) glycerol. The protein was analysed by SDS-Page.

The method measures the ability of putative ligands to modulate the interaction between the purified bacterial expressed FXR ligand binding domain (LBD) and a synthetic biotinylated peptide based on residues 676-700 of SRC-1 (LCD2, 676-700). The sequence of the peptide used was B-CPSSHSSLTERHKILHRLLQEGSPS-COOH where the N-terminus was biotinylated (B). The ligand binding domain (LBD) of FXR was expressed as fusion protein with GST in BL-21 cells using the vector pDEST15. Cells were lysed by sonication, and the fusion proteins purified over glutathione sepharose (Pharmacia) according to the manufacturers instructions. For screening of compounds for their influence on the FXR-peptide interaction, the Perkin Elmer LANCE technology was applied. This method relies on the binding dependent energy transfer from a donor to an acceptor fluorophor attached to the binding partner of interest. For ease of handling and reduction of background from compound fluorescence LANCE technology makes use of generic fluorophore labels and time resolved detection Assays were done in a final volume of 2 5µl in a 384 well plate, in a Tris-based buffer (20 mM Tris-HCl pH 7,5; 60 mM KCI, 5 mM MgCl₂; 35 ng/µl BSA), containing 20-60 ng/well recombinantly expressed FXR-LBD fused to GST, 200-600 nM N-terminally biotinylated peptide, representing SRC1 aminoacids 676-700, 200 ng/well Streptavidin-xIAPC conjugate(Prozyme) and 6-10 ng/well Eu W1024 - antiGST (Perkin Elmer). DMSO content of the samples was kept at 1%. After generation of the assay mix and diluting the potentially FXR modulating ligands, the assay was equilibrated for one hour in the dark at room temperature in FIA-plates black 384 well (Greiner). The LANCE signal was detected by a Perkin Elmer VICTOR2VTM Multilabel Counter The results were visualized by plotting the ratio between the emitted light at 665 nm and 615 nm. A basal level of FXR-peptide formation is observed in the absence of added ligand. Ligands that promote the complex formation induce a concentration-dependent increase in time-resolved fluorescent signal. Compounds which bind equally well to both monomeric FXR and to the FXR-peptide complex would be expected to give no change in signal, whereas ligands which bind preferentially to the monomeric receptor would be expected to induce a concentration-dependent decrease in the observed signal.
To assess the inhibitory potential of the compounds, EC₅₀-values were determined for example compounds as well as for comparative compounds as listed below:

### Direct reporter (DR) assay

In the direct reporter gene assay, HEK293 cells, obtained from DSMZ (German Collection of Microorganisms and Cell Cultures, Braunschweig, Germany) were stably transfected with pTRexDest30 (Invitrogen) derivative pTRexDest30-hFXR and pGL2promoter (Promega) derivative pGL2promoter-2xIBABP-RE. The full length human FXR (NM_005123) was cloned into pTRexDest30 according to the manufacturer protocol for the Gateway system (Invitrogen). The FXR response elements cccaGGGTGAaTAACCTcggggctctgtccctccaatcccaGGGTGAaTAACCTcggg were derived from the human IBABP-P promoter (Grober et al. 1999, JBC, 174, pp.29749-29754) and cloned into pGL2promoter. A stable cell line named D2IV3 was identified, which showed an 8-fold induction of luciferase activity in extracts after growing the cells in minimum essential medium (MEM) supplemented with 100nM of the FXR agonist GW4064 compared to cells grown in presence of DMSO as a vehicle. For the direct reporter gene assays, D2IV3 cells were grown in MEM with L-Glutamine and Earle's BSS supplemented with 10% fetal bovine serum, 0.1mM nonessential amino acids, 1mM sodium pyruvate, and 100units Penicilin/Streptavidin per ml at 37°C in 5% CO₂. Medium and supplements were obtained from Invitrogen. For the assay, 5x10⁵ cells were plated per well in 96well plates in 100µl per well minimum essential medium (same composition as above) incubated at 37°C in 5% CO₂. The following day the cells were >90% confluence. Compounds were prediluted in MEM and added at different concentrations to the cells with the final DMSO vehicle concentration not exceeding 0.1%. Cells were incubated for additional 16 hours before firefly luciferase activity was measured. All experiments were done in triplicates.

### Mammalian one hybrid (M1H) assay

Determination of a ligand mediated Gal4 promoter driven transactivation to quantify ligand binding mediated activation of FXR was performed as follows: The cDNA part encoding the FXR ligand binding domain was cloned into vector pCMV-BD (Stratagene) as a fusion to the yeast GAL4 DNA binding domain under the control of the CMV promoter. The amino acid boundaries of the ligand binding domain were amino acids 187-472 of Database entry NM_005123 (RefSeq). The plasmid pFR-Luc (Stratagene) was used as the reporter plasmid, containing a synthetic promoter with five tandem repeats of the yeast GAL4 binding sites, driving the expression of the Photinus pyralis (American firefly) luciferase gene as the reporter gene. In order to improve experimental accuracy the plasmid pRL-CMV (Promega) was cotransfected. pRL-CMV contains the constitutive CMV promoter, controlling the expression of the Renilla reniformis luciferase. All Gal4 reporter gene assays were done in HEK293 cells (obtained from DSMZ, Braunschweig, Germany) grown in MEM with L-Glutamine and Earle's BSS supplemented with 10% fetal bovine serum, 0.1 mM nonessential amino acids, 1mM sodium pyruvate, and 100units Penicilin/Streptavidin per ml at 37°C in 5% CO₂. Medium and supplements were obtained from Invitrogen. For the assay, 5x10⁵ cells were plated per well in 96well plates in 100µl per well MEM without Phenol Red and L-Glutamine and with Earle's BSS supplemented with 10% charcoal/dextran treated FBS (HyClone, South Logan, Utah), 0.1 mM nonessential amino acids, 2 mM glutamine, 1 mM sodium pyruvate, and 100 units Penicilin/Streptavidin per ml, incubated at 37 °C in 5% CO₂. The following day the cells were >90% confluence. Medium was removed and cells were transiently transfected using 20µl per well of a OptiMEM - polyethylene-imine-based transfection-reagent (OptiMEM, Invitrogen; Polyethyleneimine, Aldrich Cat No. 40,827-7) including the three plasmids described above. MEM with the same composition as used for plating cells was added 2-4 hours after addition of transfection mixture. Then compound stocks, prediluted in MEM were added (final vehicle concentration not exceeding 0.1 %). Cells were incubated for additional 16 hours before firefly and renilla luciferase activities were measured sequentially in the same cell extract using a Dual-Light-Luciferase-Assay system (Dyer et al., Anal Biochem 2000, (282), 158-161). All experiments were done in triplicates.
To assess the FXR agonistic potency of the example compounds as well as for reference compounds from WO2008025540, EC50-values were determined in the FRET, DR and M1H assays as listed below in Table 1:

**Table 1**

| **Compound #** | **isoxazole 5-substituent** | **FRET assay EC₅₀ (±sd)** | **DR assay EC₅₀ (±sd)** | **M1H assay EC₅₀ (±sd)** |
|---|---|---|---|---|
| Compound C | isopropyl | 11(±1.5) nM | 5.7(±1.2) | 186 |
| Compound B | isopropyl | 17(±3.6) nM | 13(±1.5) | 95(±9.9) |
| Example 1 | cyclopropyl | 8.4(±0.6) nM | 6.4(±2.3) | 56(±22) |
| Example 2 | cyclopropyl | 7.7(±1.6) nM | 6.0(±2.2) | 40(±21) |
| Example 3 | cyclopropyl | 11.5(±0.9) nM | 6 | 55 |

It can be taken from Table 1 that FXR agonists described herein exhibit increased potency towards FXR in biochemical as well as cellular *in vitro* assays compared to compounds exemplified in WO2008/025540. Such an increased potency is observed for compounds of the present invention in which the isoxazole ring of (X¹) in formula (I) carrys a cyclopropyl substituent in 5-position, or in which the triazole ring of (X²) in formula (I) of the present invention carrys a cyclopropyl substituent in 1-position.

### Determination of plasma concentration in mice

Compounds were applied perorally by gavage at 25 mg/kg each to male 8 weeks old C57BI/6J mice and plasma concentrations of the test items were determined by LC-MS/MS after time points as indicated. A solution of 2.5 mg/ml of each test item was produced by diluting them in the vehicle, 30% HPBCD (hydroxypropyl-betacyclodextrin) in 20 mM phosphate buffer pH7.0 (v/w). The solutions were stirred overnight at room temperature and heated to 60°C for 10 minutes, resulting in full solubilization. The application was performed by administrating the solution perorally to the mice, with an application volume of 10 ml/kg. For each time point three mice were used. Blood samples were obtained by sacrificing animals for each time point followed by cardiac puncture. Blood samples were treated with Li-heparin during collection procedure and stored on ice until centrifugation at 645 g (5 min, 4°C). Plasma was harvested and kept at -20°C until being assayed. To 50 µl of mouse plasma sample 6 µl acetonitrile containing an internal standard was added. Samples were vigorously shaken and centrifuged for 10 minutes at 6000 g and 20°C. An aliquot of the particle-free supernatant was transferred to 200 µl sampler vials and subsequently subjected to LC MS/MS for quantification. Plasma concentrations at various time points were determined and are plottet against sampling times as shown in Figure 1.
Unexpectedly, it was found that FXR agonists described herein exhibit increased plasma levels after oral administration *in vivo* as compared to known FXR agonists described in the prior art. Such increased plasma levels are observed for compounds of the present invention in which substituents L and A of the central phenyl ring in formula (I) are in a 1,4 disposition.

## Claims

1. A compound of formula (I) 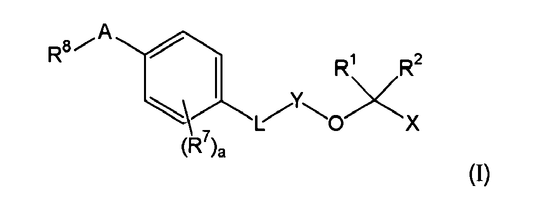 or an enantiomer, diastereomer, tautomer, solvate or pharmaceutically acceptable salt thereof,
wherein
R¹ and R² are independently from each other selected from hydrogen, fluorine and C₁-C₃ alkyl;
X is 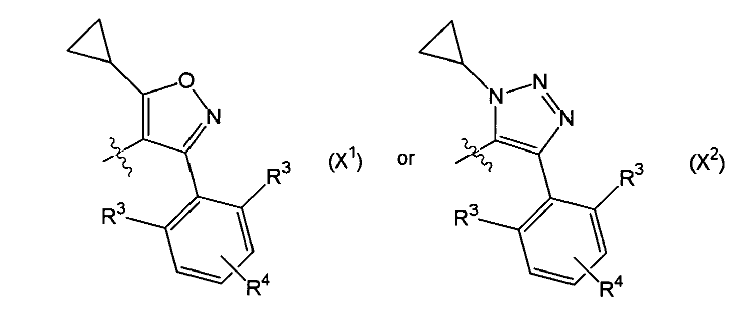
R³ is independently selected from hydrogen, halogen, C₁-C₃ alkyl and O-C₁-C₃ alkyl wherein each alkyl is unsubstituted or substituted with one or more fluorine atoms;
R⁴ is hydrogen or fluorine;
Y is selected from 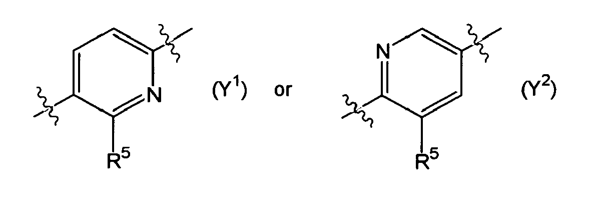
R⁵ is halogen, cyano, C₁-C₃ alkyl, C₂-C₃ alkenyl, O-C₁-C₃ alkyl or C₃-C₆ cycloalkyl, wherein each alkyl, alkenyl and cycloalkyl is unsubstituted or substituted with one to five substituents R¹⁰;
L is -CH₂-N(R⁶)-, -CH₂O- or O-CH₂;
R⁶ is C₁-C₃-alkyl, or C₃-C₅ cycloalkyl, wherein each alkyl or cycloalkyl is unsubstituted or substituted with 1-5 fluorine atoms;
R⁷ is halogen, C₁-C₆ alkyl, C₃-C₆ cycloalkyl, C₂-C₆ alkenyl, C₂-C₆ alkynyl, cyano, O-C₁-C₆ alkyl or O-C₃-C₆ cycloalkyl, wherein each alkyl, alkenyl, alkynyl and cycloalkyl is unsubstituted or substituted with one to five substituents R¹⁰;
A is a bond, CH₂, CHCH₃, C(CH₃)₂, -CH₂O-, -CH₂CH₂- or -CH=CH-;
R⁸ is COOR⁹, CONHR⁹, C(O)NHSO₂R⁹ or tetrazole which is connected to A via the C-atom;
R⁹ is hydrogen, C₁-C₆-alkyl, C₃-C₆-cycloalkyl or a 4-7-membered heterocyclic ring, wherein the alkyl, cycloalkyl and heterocyclyl groups are unsubstituted or substituted with one to three substituents R¹⁰;
R¹⁰ is independently selected from hydrogen, halogen, cyano, nitro, azido, =O, =S, C₁-C₆ alkyl, C₂-C₆ alkenyl, C₂-C₆ alkynyl, C₃-C₆ cycloalkyl, 4-7 membered heterocyclyl, aryl, heteroaryl, NR¹¹R¹², NR¹¹SO₂R¹², NR¹¹C(O)OR¹², NR¹¹C(O)R¹², -OR¹¹, OC(O)R¹¹, SO₂R¹¹, SO₃R¹¹, SO₂NR¹¹R¹², C(O)R¹¹, C(O)OR¹¹ and C(O)NR¹¹R¹²;
R¹¹ and R¹² are independently from each other selected from hydrogen, C₁-C₆ alkyl and C₃-C₆ cycloalkyl, wherein each alkyl or cycloalkyl may be unsubstituted or substituted with one to five fluorines and/or one to three substituents selected from OH, OCH₃, OCH₂F, OCHF₂, OCF₃, =O, NH₂, NHCH₃ and N(CH₃)₂;
a is 0, 1, 2 or 3.

2. The compound according to claim 1, wherein
R¹ and R² are both hydrogen.

3. The compound according to claim 1 or 2, wherein
X is (X¹).

4. The compound according to any of claims 1 to 3, wherein
R³ is hydrogen, fluorine, chlorine, CF₃ or OCF₃.

5. The compound according to any of claims 1 to 4, wherein
L is -CH₂-N(R⁶)-, and
R⁶ is C₁-C₃-alkyl.

6. The compound according to any of claims 1 to 5, wherein
A is a bond.

7. The compound according to any of claims 1 to 6, wherein
R⁷ is C₁-C₆ alkyl or O-C₁-C₆ alkyl.

8. The compound according to any of claims 1 to 7, wherein
R⁸ is COOR⁹.

9. The compound according to any of claims 1 to 8 as a medicament.

10. A pharmaceutical composition comprising at least one compound according to any of claims 1 to 8 and at least one pharmaceutically acceptable excipient and/or carrier.

11. A compound according to any of claims 1 to 8 for the prophylaxis and/or treatment of diseases mediated by FXR.

12. The compound according to claim 11 for the prophylaxis and/or treatment of chronic intrahepatic or some forms of extrahepatic cholestatic conditions, or liver fibrosis resulting from chronic cholestatic conditions or acute intraheptic cholestatic conditions.

13. The compound according to claim 11 for the prophylaxis and/or treatment of obstructive or chronic inflammatory disorders that arise out of improper bile composition.

14. The compound according to claim 11 for the prophylaxis and/or treatment of gastrointestinal conditions with a reduced uptake of dietary fat and fat-soluble dietary vitamins.

15. The compound according to claim 11 for the prophylaxis and/or treatment of Inflammatory Bowel Diseases.

16. The compound according to claim 11 for the prophylaxis and/or treatment of lipid and lipoprotein disorders.

17. The compound according to claim 11 for the prophylaxis and/or treatment of Type II Diabetes and clinical complications of Type I and Type II Diabetes.

18. The compound according to claim 11 for the prophylaxis and/or treatment of conditions and diseases which result from chronic fatty and fibrotic degeneration of organs due to enforced lipid and specifically triglyceride accumulation and subsequent activation of profibrotic pathways.

19. The compound according to claim 11 for the prophylaxis and/or treatment of obesity and metabolic syndrome (combined conditions of dyslipidemia, diabetes and abnormally high body-mass index).

20. The compound according to claim 11 for the prophylaxis and/or treatment of acute myocardial infarction, acute stroke, or thrombosis which occur as an endpoint of chronic obstructive atherosclerosis.

21. The compound according to claim 11 for the prophylaxis and/or treatment of persistant infections by intracellular bacteria or parasitic protozoae.

22. The compound according to claim 11 for the prophylaxis and/or treatment of non-malignant hyperproliferative disorders and malignant hyperproliferative disorders.

23. The compound according to claim 11 for the prophylaxis and/or treatment of liver steatosis and associated syndromes, cholestatic and fibrotic effects that are associated with alcohol-induced cirrhosis or with viral-borne forms of hepatitis.
